(19) Europäisches Patentamt
European Patent Office
Office européen des brevets

(11) **EP 1 941 862 A1**

(12) **EUROPEAN PATENT APPLICATION**
published in accordance with Art. 153(4) EPC

(43) Date of publication:
**09.07.2008 Bulletin 2008/28**

(21) Application number: **06797061.6**

(22) Date of filing: **30.08.2006**

(51) Int Cl.:
**A61K 8/60** (2006.01)     **A61J 3/10** (2006.01)
**A61K 9/20** (2006.01)     **A61K 47/26** (2006.01)
**A61P 1/02** (2006.01)     **A61Q 11/00** (2006.01)

(86) International application number:
**PCT/JP2006/317088**

(87) International publication number:
**WO 2007/026755 (08.03.2007 Gazette 2007/10)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **31.08.2005 JP 2005252624**

(71) Applicant: **Ezaki Glico Co., Ltd.**
**Osaka-shi,**
**Osaka 555-8502 (JP)**

(72) Inventors:
• **IOKA, Toshiyuki**
**Nishinomiya-shi, Hyogo 6638113 (JP)**

• **YOSHIMATSU, Daisuke**
**Amagasaki-shi, Hyogo 6610014 (JP)**
• **SHIRAISHI, Koso**
**Toyonaka-shi, Osaka 5600013 (JP)**
• **SUGIMURA, Shinji**
**Ibaraki-shi, Osaka 5670012 (JP)**

(74) Representative: **UEXKÜLL & STOLBERG**
**Patentanwälte**
**Beselerstrasse 4**
**22607 Hamburg (DE)**

(54) **TABLET FOR REMOVAL OF TONGUE COATING**

(57)     A tablet for removing tongue coating, wherein the tablet contains highly soluble saccharide and lowly soluble saccharide, the highly soluble saccharide and the lowly soluble saccharide are water-soluble, of water-soluble saccharides contained as the primary component in the tablet, the highly soluble saccharide has the highest solubility in water at 37°C, the highly soluble saccharide is a saccharide having the highest solubility in water at 37°C among the water-soluble saccharides contained in the tablet as the main components, the lowly soluble saccharide is a saccharide having the lowest solubility in water at 37°C among the water-soluble saccharides contained in the tablet as the main components, and the difference between the solubility of the highly soluble saccharide in water at 37°C and the solubility of the lowly soluble saccharide in water at 37°C is 10 % by weight or more.

EP 1 941 862 A1

**Description**

TECHNICAL FIELD

The present invention relates to a tablet for removing tongue coating and a production method thereof.

BACKGROUND ART

Causative substances of halitosis are volatile sulfur compounds (hereinafter abbreviated as VSC) such as hydrogen sulfide ($H_2S$), methyl mercaptan ($CH_3SH$), dimethyl sulfide (($CH_3$)$_2$S) (Non-Patent Document 1). Even at 0.3 ppm, which is the ordinary halitosis concentration, exposure to $H_2S$ for a long time will cause nausea, insomnia and breathing disorders. The main constituents of physiological halitosis originate from tongue coating composed of desquamated epithelium cells, bacteria, food residue, blood cells or the like adhered to the back portion of the dorsum of the tongue (Non-Patent Documents 2 to 4), and are primarily VSC produced by bacterial putrefactive action at said portion. On the other hand, causes of pathological halitosis due to periodontal disease are consistent in principle with those of physiological halitosis (Non-Patent Document 5), and about 60% of all VSCs are produced from tongue coating (Non-Patent Document 6). Since people often keep away from those having halitosis, it can be said that halitosis wouldbe an inhibitory factor for a healthy social life. Therefore, it can be said that halitosis is a disease damaging to both the mind and body of humans.

For treatment of halitosis derived from the oral cavity, removing tongue coating by cleaning the tongue is indispensable, and tongue cleaning using an instrument such as a tongue brush is instructed. However, it is necessary to be sufficiently careful of mechanical irritation to the tongue caused by such an instrument. It is required that the instrument is used in a delicate manner.

Therefore, it is desired to provide a method for removing tongue coating, which neither requires an instrument nor delicate usage, but is effective at removing tongue coating, is simple, and is comfortable to use.

The present inventors previously made an invention directed to a composition for removing or preventing halitosis which contains a compound having an action of removing halitosis in an amount effective for removing halitosis, and a composition for removing tongue coating which contains a plant-derived preparation wherein the plant-derived preparation contains a compound having an effect of removing tongue coating (Patent Document 1). In this invention, an effect of removing tongue coating was obtained using a compound having an action of removing tongue coating, and is based on a chemical method. On the other hand, the present invention is based on physical removal utilizing roughness on a tablet surface. Thus, the principles are entirely different from each other. Further, with a troche described in Examples 4 and 5 of Patent Document 1, only one type of saccharide is used for the base, and therefore, roughness generated on the tablet surface by sucking are insufficient in order to exert an action of removing tongue coating.

[Patent Document 1] WO03090704 pamphlet
[Non-Patent Document 1] Tonzetich J., "Direct gas chromatographic analysis of sulphur compounds in mouth air in man.", Archs. Oral Biol., 16:587-597, 1971
[Non-Patent Document 2] Tonzetich J. , Coil J.M. and Ng W. , "Gas chromatographic method for trapping and detection of volatile organic compounds from human mouth air.", J. Clin. Dent. 11:79-82, 1991
[Non-Patent Document 3] Tonzetich J., Eigan E., King W.J. et al., "Volatility as a factor in the inability of certain amines and indole to increase the odour of saliva.", Archs. Oral Biol. 12:1167-1175, 1967
[Non-Patent Document 4] Tonzetich J. and McBride B.C., "Characterization of volatile sulfur production by pathogenic and non-pathogenic strains of oral Bacteroides.", Archs. Oral Biol. 26:963-969, 1981
[Non-Patent Document 5] Yaegaki Ken, Miyazaki Hideo, and Kawaguchi Yoko, "Rinsho-Ka No Tame No Koshu Chiryo Guideline (Guideline for halitosis treatment for clinicians)", Quintessence Publishing, Tokyo, 2000
[Non-Patent Document 6] Yaegaki K. and Sanaga K., "Biochemical and clinical factors influencing oral malodor in periodontal patients.", J. Periodontol. 63: 783-789, 1992
[Non-Patent Document 7] MORIYA Toshiki, KISHI Mitsuo, AIZAWA Fumie, et al., "Zettai Score Ni Yoru Koshu Screening No Yukosei Ni Kansuru Kenkyu (Tongue Coating Score as a Screening Test for Oral Malodor)", Journal of Dental Health 52: 12-21, 2002

DISCLOSURE OF THE INVENTION

PROBLEMS TO BE SOLVED BY THE INVENTION

The present invention intends to solve the aforementioned problems. An object of the present invention is to provide a tablet for removing tongue coating which is effective at removing tongue coating, is simple, and is comfortable to use, and a production method thereof.

MEANS TO SOLVE THE PROBLEMS

The present inventors considered that, when a tablet, candy or the like is taken by sucking, if the surface thereof is rough and there are concavities and convexities, the tongue coating is scraped and removed by contact of the tablet, candy or the like with the dorsum of the tongue. The present inventors further considered that by sucking a tablet, candy or the like, saliva secretion is accelerated thereby a washout action is obtained, and an effect of removing tongue coating is obtained. Therefore, the present inventors developed a tablet which forms concavities and convexities on the surface when taking it by sucking. In particular, the present inventors developed a large tablet of sugarless type contemplated to remove tongue coating physically by taking it by sucking.

As a result of diligent studies in order to solve the above-mentioned problems, the present inventors have found that an effect of removing tongue coating and halitosis reduction effects can be obtained by sucking a tablet which forms concavities and convexities on the surface when taking it by sucking. Based on these findings, the present inventors completed the present invention.

The tablet according to the present invention is a tablet for removing tongue coating, wherein the tablet contains a highly soluble saccharide and a lowly soluble saccharide,
the highly soluble saccharide and the lowly soluble saccharide are water-soluble,
the highly soluble saccharide is a saccharide having the highest solubility in water at 37°C among the water-soluble saccharides contained in the tablet as the main components,
the lowly soluble saccharide is a saccharide having the lowest solubility in water at 37°C among the water-soluble saccharides contained in the tablet as the main components, and
the difference between the solubility of the highly soluble saccharide in water at 37°C and the solubility of the lowly soluble saccharide in water at 37°C is 10 % by weight or more.

The tablet of the present invention is a tablet for removing tongue coating, wherein the tablet contains a highly soluble saccharide and a lowly soluble saccharide,
the highly soluble saccharide and the lowly soluble saccharide are water-soluble,
the highly soluble saccharide is a saccharide having the highest solubility in water at 37°C among the water-soluble saccharides present in the tablet at 10 % by weight or more,
the lowly soluble saccharide is a saccharide having the lowest solubility in water at 37°C among the water-soluble saccharides present in the tablet at 10 % by weight or more, and
the difference between the solubility of the highly soluble saccharide in water at 37°C and the solubility of the lowly soluble saccharide in water at 37°C is 10 % by weight or more.

The tablet of the present invention is a tablet for removing tongue coating, wherein the tablet contains a highly soluble saccharide and a lowly soluble saccharide,
the highly soluble saccharide and the lowly soluble saccharide are water-soluble,
the highly soluble saccharide is a saccharide having the highest solubility in water at 37°C among the water-soluble saccharides present in the tablet at 20 % by weight or more,
the lowly soluble saccharide is a saccharide having the lowest solubility in water at 37°C among the water-soluble saccharides present in the tablet at 20 % by weight or more, and
the difference between the solubility of the highly soluble saccharide in water at 37°C and the solubility of the lowly soluble saccharide in water at 37°C is 10 % by weight or more.

In one embodiment, the above-mentioned difference is 15 % by weight or more.

In one embodiment, the above-mentioned difference is 20 % by weight or more.

In one embodiment, the above-mentioned difference is 25 % by weight or more.

In one embodiment, the above-mentioned difference is 30 % by weight or more.

In one embodiment, the above-mentioned difference is 35 % by weight or more.

In one embodiment, the solubility of the above-mentioned highly soluble saccharide in water at 37°C is 50 % by weight or more.

In one embodiment, the above-mentioned highly soluble saccharide is selected from the group consisting of glucose, maltitol, sorbitol, xylitol, erythritol, maltose, sucrose, fructose, and cellobitol.

In one embodiment, the solubility of the above-mentioned lowly soluble saccharide in water at 37°C is 5 % by weight or more and 40 % by weight or less.

In one embodiment, the above-mentioned lowly soluble saccharide is selected from the group consisting of palatinit, mannitol, lactose, and palatinose.

In one embodiment, the tablet further contains another water-soluble saccharide in addition to said highly soluble saccharide and said lowly soluble saccharide.

In one embodiment, the above-mentioned highly soluble saccharide is sorbitol, the above-mentioned lowly soluble saccharide is palatinit, and the above-mentioned another water-soluble saccharide is maltitol.

In one embodiment, the above-mentioned tablet takes 5 minutes or more for disintegration in the oral cavity.

In one embodiment, the above-mentioned tablet has a hardness of 15 kgf or more.

In one embodiment, the above-mentioned tablet further contains a binder.

In one embodiment, the above-mentioned tablet further contains an enzyme.

In one embodiment, the above-mentioned enzyme is a protease.

In one embodiment, the above-mentioned enzyme is bromelain or papain.

A production method of the present invention is a production method of a tablet for removing tongue coating, comprising the steps of:

mixing a highly soluble saccharide and a lowly soluble saccharide to obtain a mixture; and
compressing the mixture to obtain a tablet, wherein
the highly soluble saccharide and the lowly soluble saccharide are water-soluble saccharides,
the highly soluble saccharide is a saccharide having the highest solubility in water at 37°C among the water-soluble saccharides contained in the tablet as the main components,
the lowly soluble saccharide is a saccharide having the lowest solubility in water at 37°C among the water-soluble saccharides contained in the tablet as the main components, and
the difference between the solubility of the highly soluble saccharide in water at 37°C and the solubility of the lowly soluble saccharide in water at 37°C is 10 % by weight or more.

A production method of the present invention is a production method of a tablet for removing tongue coating, comprising the steps of:

mixing a highly soluble saccharide and a lowly soluble saccharide to obtain a mixture; and
compressing the mixture to obtain a tablet, wherein
the highly soluble saccharide and the lowly soluble saccharide are water-soluble saccharides,
the highly soluble saccharide is a saccharide having the highest solubility in water at 37°C among the water-soluble saccharides present in the tablet at 10 % by weight or more,
the lowly soluble saccharide is a saccharide having the lowest solubility in water at 37°C among the water- soluble saccharides present in the tablet at 10 % by weight or more, and
the difference between the solubility of the highly soluble saccharide in water at 37°C and the solubility of the lowly soluble saccharide in water at 37°C is 10 % by weight or more.

A production method of the present invention is a production method of a tablet for removing tongue coating, comprising the steps of:

mixing a highly soluble saccharide and a lowly soluble saccharide to obtain a mixture; and
compressing the mixture to obtain a tablet, wherein
the highly soluble saccharide and the lowly soluble saccharide are water-soluble saccharides,
the highly soluble saccharide is a saccharide having the highest solubility in water at 37°C among the water-soluble saccharides present in the tablet at 20 % by weight or more,
the lowly soluble saccharide is a saccharide having the lowest solubility in water at 37°C among the water- soluble saccharides present in the tablet at 20 % by weight or more, and
the difference between the solubility of the highly soluble saccharide in water at 37°C and the solubility of the lowly soluble saccharide in water at 37°C is 10 % by weight or more.

EFFECT OF THE INVENTION

By taking the tablet of the present invention by sucking, the amount of tongue coating is reduced. In other words, an effect of physically removing attached tongue coating is obtained by a washout action by contact of the tablet with the dorsum of the tongue, and by accelerating saliva secretion. Therefore, halitosis reduction effects are obtained by taking the tablet of the present invention by sucking.

BRIEF DESCRIPTION OF THE DRAWINGS

FIG. 1 shows a solubility curve based on solubility at various temperatures of typical water-soluble saccharides.
FIG. 2 shows the judgmental rating reference diagram for the amount of tongue coating.
FIG. 3 (A) shows a photograph of the tablet surface of Example 1 three minutes after immersion into water, and FIG 3 (B) shows a photograph of the tablet surface of Example 1 seven minutes after immersion into water.

FIG. 4 (A) shows a photograph of the tablet surface of Comparative Example 1 three minutes after immersion into water, and FIG 4 (B) shows a photograph of the tablet surface of Comparative Example 1 seven minutes after immersion into water.

FIG. 5 (A) shows a photograph of the tablet surface of Comparative Example 2 three minutes after immersion into water, and FIG 5 (B) shows a photograph of the tablet surface of Comparative Example 2 seven minutes after immersion into water.

FIG. 6 (A) shows a photograph of the tablet surface of Comparative Example 3 three minutes after immersion into water, and FIG 6 (B) shows a photograph of the tablet surface of Comparative Example 3 seven minutes after immersion into water.

FIG. 7 (A) shows a photograph of the tablet surface of Comparative Example 4 three minutes after immersion into water, and FIG 7 (B) shows a photograph of the tablet surface of Comparative Example 4 seven minutes after immersion into water.

FIG. 8 is a graph showing changes in tongue coating score due to taking the tablet. Mark [*] indicates that $p < 0.01$. Tongue coating score was determined by visual inspection evaluation.

FIG. 9 (A) is a graph showing the reduction ratio of tongue coating score by taking the tablet of any of Example 3 through Example 6. FIG. 9 (B) is a graph showing the reduction ratio of tongue coating score by taking the tablet of any of Comparative Example 1 through Comparative Example 4. Tongue coating score was determined by visual inspection evaluation.

FIG. 10 is a graph showing change ratios of $H_2S$ concentration by taking the tablet of Example 7 or Example 8.

FIG. 11 is a graph showing change ratios of $CH_3SH$ concentration by taking the tablet of Example 7 or Example 8.

FIG. 12 is a graph showing change ratios of tongue coating score after taking the tablet of Example 9 or Example 10.

BEST MODE FOR CARRYING OUT THE INVENTION

The present invention will be described hereinafter in detail.

A tablet for removing tongue coating and production methods thereof are provided by the present invention.

As used herein, the "tongue coating" refers to substances adhered onto the upper surface of the tongue (i.e., the dorsum of the tongue) and comprises epithelial remnants, food particles, bacteria, or the like. Tongue coating is a primary cause of halitosis in a similar fashion to dental plaque, periodontal pocket, and saliva.

As used herein, "for removing tongue coating" refers to those used primarily for the purpose of removing tongue coating.

As used herein, "tablet" refers to those produced by compressing tablet materials into a certain shape. Such tablet is referred to as compressed tablet named after the production method thereof. As used herein, the tablets may be those for food applications or pharmaceutical applications (i. e. , as tablets). Examples of tablets for food application include a tablet as confection referred to as a tablet candy, and a tablet as health food (e.g., supplements). As used herein, those having a hole at the center part thereof are referred particularly to as a troche.

(1. Materials for a tablet for removing tongue coating)

(1.1 Saccharides)

As used herein, "saccharide" refers to a compound composed of carbon, hydrogen, and oxygen. Saccharide includes saccharide and carbohydrate. Saccharide is preferably a sweet compound. Saccharide is preferably selected from the group consisting of monosaccharide, disaccharide, oligosaccharide, sugar alcohol, glucose syrup, degraded starch, and water-soluble dietary fiber, and more preferably, is selected from the group consisting of monosaccharide, disaccharide, oligosaccharide, and sugar alcohol. Any saccharide commercially available in the art may be used as the saccharide.

The saccharide used in the tablet of the present invention is water-soluble. As used herein, "water-soluble" refers to a substance having solubility in water at 37°C at 1 % by weight or more. Solubility of saccharide used in the present invention is preferably about 2 % by weight or more, more preferably about 3 % by weight or more, further preferably about 4 % by weight or more, particularly preferably about 5 % by weight or more.

Examples of water-soluble saccharide include monosaccharides such as fructose, glucose, xylose and the like; disaccharides such as sucrose, maltose, lactose, palatinose, trehalose and the like; sugar alcohol such as palatinit (also referred to as reducedpalatinose), maltitol, sorbitol, erythritol, xylitol, lactitol, mannitol and the like; and degraded starch such as glucose syrup, dextrin, oligosaccharide and the like.

Among the water-solublesaccharides, non-cariogenic saccharides are preferable, palatinit, palatinose, maltitol, sorbitol, erythritol, xylitol, trehalose, lactitol, and mannitol are preferable.

For example, quality of taste of palatinit is similar to that of sucrose and is free from any abnormal taste. Further, palatinit has a feature that non-sticky candy is obtained even if a candy is produced using only palatinit as the excipient.

Quality of the taste of palatinose is similar to that of sucrose and is free from any abnormal taste. Further, palatinose has the following features : it is a non-cariogenic saccharide; it is gently digested and absorbed in the body to produce energy, and does not cause a sudden change in blood sugar level and insulin secretion; it is a low sweetness intensity substance which is resistant to acid and has excellent quality of taste.

The quality of the taste of maltitol is slightly "indistinct" as compared to that of sucrose, and the abnormal taste of maltitol is slightly irritating to the throat. Maltitol is non-hygroscopic, like sucrose.

The quality of taste of sorbitol is slightly "indistinct" as compared to that of sucrose, and the abnormal taste of sorbitol is slightly irritating to the throat. Sorbitol has excellent permeability, moisture retention, and improvement of shelf-life.

Erythritol has a unique sweet taste, and the sweet taste of it appears quickly. Erythritol has a great endothermal action (-43 cal/g) when dissolved and therefore, the feeling of coolness is significant. Erythritol also has a feature that it is unlikely to results in diarrhea as compared to other sugar alcohol.

Xylitol has a unique sweet taste. Xylitol also has a great endothermal action (-37 cal/g) when dissolved and therefore, the feeling of coolness is significant. Xylitol is evaluated as having better taste compared to erythritol. Further, the saliva secretion accelerating action of xylitol is stronger than those of other sugar alcohols.

The quality of taste of lactitol includes a sweet taste and is free from abnormal taste.

The water-soluble saccharide is preferably an excipient which is not assimilated by oral bacteria. Examples of water-soluble saccharides which are not assimilated by oral bacteria include palatinit, palatinose, maltitol, sorbitol, erythritol, xylitol, trehalose, lactitol, and mannitol. If the water-soluble saccharide is assimilated by oral bacteria, a cause of increasing oral bacteria is generated by taking a tablet containing this water-soluble saccharide. Such a water-soluble saccharide can act negatively against an action of removing tongue coating. Therefore, water-soluble saccharide is preferably a substance which cannot be assimilated by oral bacteria. One type of water-soluble saccharide may be used solely or a plurality of types of water-soluble saccharide may be used in mixture. When a plurality of types of water-soluble saccharides are used, the weight of water-soluble saccharides which cannot be assimilated by oral bacteria (non-cariogenic saccharides) is preferably about 20 % by weight or more, about 30 % by weight or more, about 40 % by weight or more, about 50 % by weight or more, about 60 % by weight or more, about 70 % by weight or more, about 80 % by weight or more, and about 90 % by weight or more of the total weight of water-soluble saccharides. Formulation of a troche can be in accordance with the formulation known in the art.

Table 1 below shows the solubility of typical water-soluble saccharides in water at 37°C.

(Table 1)

| Saccharides | Solubility in water at 37°C |
|---|---|
| Fructose | 84 |
| Glucose | 67 |
| Sucrose | 68 |
| Maltose | 52 |
| Lactose | 24 |
| Palatinit | 37 |
| Palatinose | 40 |
| Maltitol | 63 |
| Sorbitol | 74 |
| Erythritol | 44 |
| Xylitol | 72 |
| Trehalose | 52 |
| Lactitol | 70 |
| Mannitol | 23 |

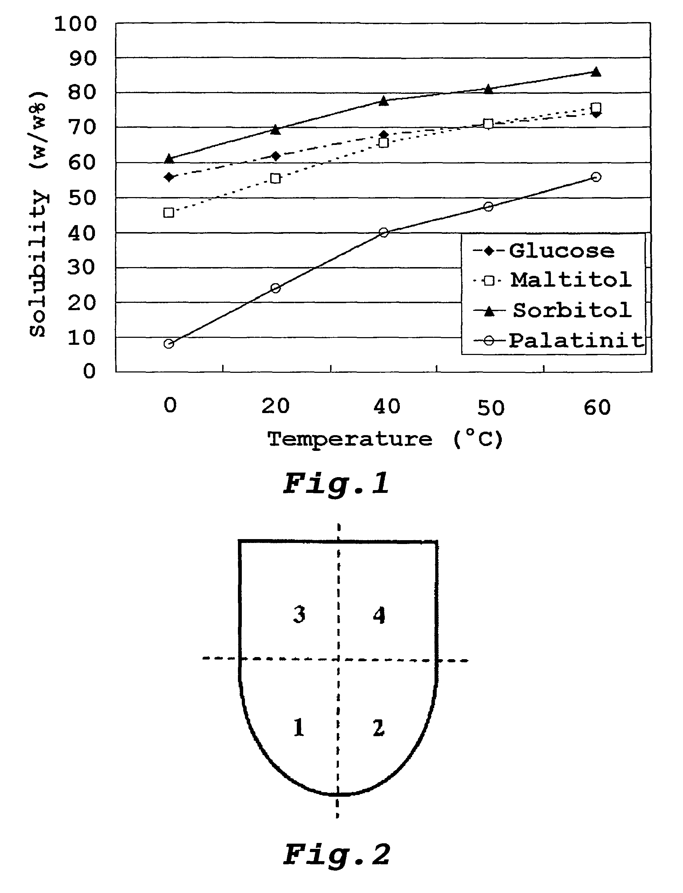

Further, FIG. 1 shows solubility of typical water-soluble saccharides at various temperatures.

(1.1.1 Highly soluble saccharide and lowly soluble saccharide)

The tablet for removing tongue coating of the present invention contains highly soluble saccharide and lowly soluble saccharide.

Highly soluble saccharide and lowly soluble saccharide are both water-soluble saccharides. Since solubility of highly soluble saccharide is higher than those of lowly soluble saccharide, those saccharides are referred to as "high solubility" saccharides. Since solubility of lowly soluble saccharide is lower than those of highly soluble saccharide, those saccharides are referred to as "low solubility" saccharides.

The highly soluble saccharide is a saccharide having the highest solubility in water at 37°C among the water-soluble saccharides contained in the tablet for removing tongue coating of the present invention, as the main components, preferably present at 10 % by weight or more, more preferably at 20 % by weight or more. The lowly soluble saccharide is a saccharide having the lowest solubility in water at 37°C among the water-soluble saccharides contained in the tablet for removing tongue coating of the present invention, as the main components, preferably present at 10 % by weight or more, more preferably at 20 % by weight or more.

As used herein, "contained as amain component" means that the component is contained preferably at about 5 % by weight or more, more preferably at about 10 % by weight or more, further preferably at about 15 % by weight or more, and most preferably at about 20 % by weight or more.

The difference between the solubility of highly soluble saccharide in water at 37°C and the solubility of lowly soluble saccharide in water at 37°C is about 10 % by weight or more, preferably about 15 % by weight or more, more preferably about 20 % by weight or more, further preferably about 25 % by weight or more, further preferably about 30 % by weight or more, and most preferably about 35 % by weight or more. The greater the difference, the more easily are formed concavities and convexities on the surface of the tablet, and thus is preferable. There is no particular upper limit for the difference between the solubility of highly soluble saccharide in water at 37°C and the solubility of lowly soluble saccharide in water at 37°C. The difference is normally about 90 % by weight or less, but the difference can be about 85 % by weight or less, about 80 % by weight or less, about 75 % by weight or less, about 70 % by weight or less, about 65 % by weight or less, about 60 % by weight or less, about 55 % by weight or less, and about 50 % by weight or less.

For example, when a tablet for removing tongue coating of the present invention contains about 50 % by weight of water-soluble saccharide $X_1$ having a solubility of about 50 % by weight in water at 37°C, and about 50 % by weight of water-soluble saccharide $X_2$ having a solubility of about 30 % by weight in water at 37°C, and contains no other water-soluble saccharides, $X_1$ is the highly soluble saccharide and $X_2$ is the lowly soluble saccharide.

For example, if a tablet for removing tongue coating of the present invention contains about 30 % by weight of water-soluble saccharide $X_3$ having a solubility of about 50 % byweight inwater at 37°C, about 30 % by weight of water-soluble saccharide $X_4$ having a solubility of about 40 % by weight in water at 37°C, and about 30 % by weight of water-soluble saccharide $X_5$ having a solubility of about 30 % by weight in water at 37°C, and contains no other water-soluble saccharides, $X_3$ is the highly soluble saccharide and $X_5$ is the lowly soluble saccharide.

For example, if a tablet for removing tongue coating of the present invention contains about 25 % by weight of water-soluble saccharide $X_6$ having a solubility of about 50 % by weight in water at 37°C, about 25 % by weight of water-soluble saccharide $X_7$ having a solubility of about 40 % by weight in water at 37°C, about 25 % by weight of water-soluble saccharide $X_8$ having a solubility of about 30 % by weight in water at 37°C, and about 25 % by weight of water-soluble saccharide $X_9$ having a solubility of about 20 % by weight in water at 37°C, and contains no other water-soluble saccharides, $X_6$ is the highly soluble saccharide and $X_9$ is the lowly soluble saccharide.

Even if a water-soluble saccharide is contained in the tablet for tongue coating in a content less than 10 % by weight, the saccharide is not classified as the highly soluble saccharide or lowly soluble saccharide referred to herein. For example, when a tablet for removing tongue coating of the present invention contains about 5 % by weight of water-soluble saccharide $X_{10}$ having a solubility of about 50 % by weight in water at 37°C, about 35 % by weight of water-soluble saccharide $X_{11}$ having a solubility of about 40 % by weight in water at 37°C, about 35 % by weight of water-soluble saccharide $X_{12}$ having a solubility of about 30 % by weight in water at 37°C, and about 5 % by weight of water-soluble saccharide $X_{13}$ having a solubility of about 20 % by weight in water at 37°C, and contains no other water-soluble saccharides, $X_{11}$ is the highly soluble saccharide and $X_{12}$ is the lowly soluble saccharide.

It must be noted that, for example, in the case of a tablet that contains about 2 % by weight of each of saccharides $Y_1$ to $Y_{20}$ having a solubility of about 50 % by weight to about 60 % by weight in water at 37°C, and about 40 % by weight of saccharide $Y_{21}$ having solubility of about 30 % by weight in water at 37°C, this tablet does not literally fall within the tablet of the present invention. However, those skilled in the art can readily understand that such a tablet would exert the equivalent performances as those of the tablet of the present invention. This is similarly considered for cases other than the above-mentioned case, where plurality types of water-soluble saccharides are used.

When the difference between the solubility of the highly soluble saccharide in water at 37°C and the solubility of the lowly soluble saccharide in water at 37°C is 10 % by weight or more, then the solubility of the highly soluble saccharide in water at 37°C may be low, for example, about 30 % by weight. When the difference between the solubility of the highly soluble saccharide in water at 37°C and the solubility of the lowly soluble saccharide in water at 37°C is 10 % by weight or more, the solubility of the highly soluble saccharide in water at 37°C may be high, for example, about 60 % by weight.

If the ease of formation of concavities and convexities when a tablet is sucked in the oral cavity, retention time of the tablet in the oral cavity, and the like are taken into consideration, the solubility of highly soluble saccharide in water at 37°C is preferably about 50 % by weight or more, more preferably about 55 % by weight or more, and further preferably about 60 % by weight or more, preferably about 90 % by weight or less, more preferably about 85 % by weight or less, and further preferably about 80 % by weight or less. The highly soluble saccharide is preferably selected from the group consisting of glucose, maltitol, sorbitol, xylitol, erythritol, maltose, sucrose, fructose, and cellobitol, more preferably selected from the group consisting of maltitol, sorbitol, xylitol, and erythritol, and further preferably selected from the group consisting of maltitol and sorbitol.

If the ease of formation of concavities and convexities when a tablet is sucked in the oral cavity, retention time of the tablet in the oral cavity and the like are taken into consideration, the solubility of lowly soluble saccharide in water at 37°C is preferably about 5 % by weight or more, more preferably about 10 % by weight or more, and further preferably about 15 % by weight or more, preferably about 40 % by weight or less, more preferably about 35 % by weight or less, and further preferably about 30 % by weight or less. The lowly soluble saccharide is preferably selected from the group consisting of palatinit, mannitol, lactose, and palatinose, more preferably selected from the group consisting of palatinit and mannitol.

As used herein, "solubility in water at 37°C " refers to a concentration of a specific solute when it is dissolved in water at 37°C at a maximum. The solubility can be determined by a method well-known to those skilled in the art. For example, solubility (% by weight) can be determined as follows. A solute is dissolved gradually in water of weight $W_1$ at 37°C. When the largest amount of the solute is dissolved in the water, the weight of the solution is $W_2$. The weight of the dissolved solute $W_3$ (= $W_2$ - $W_1$) is divided by the weight of the solution $W_2$, and multiplied by 100 (i.e., converted to a percentage).

In this specification, solubility in water at 37°C is used for explanation of the present invention as a standard. This is because since the body temperature of the human who takes a tablet for removing tongue coating is about 37°C, there is no significant difference between the solubility in water and the solubility in the oral cavity, and therefore, behaviors similar to dissolution properties of saccharides in the oral cavity can be observed.

Both the weight of the highly soluble saccharide and weight of the lowly soluble saccharide as used in the present invention are, based on tablet weight, about 20 % by weight or more, each independently more preferably about 25 % by weight or more, further preferably about 30 % by weight or more, even more preferably about 35 % by weight or more, particularly preferably about 40 % by weight or more, especially preferably about 45 % by weight or more, and most preferably about 50 % by weight or more.

Although there is no limitation on a ratio of weight of highly soluble saccharide to weight of lowly soluble saccharide, it is normally within a range from 1:4 to 4:1. There are cases where weight of highly soluble saccharide is lower than weight of lowly soluble saccharide, and there are cases where weight of highly soluble saccharide is higher than weight of lowly soluble saccharide. In a particular case, it is preferable that the weight of highly soluble saccharide is higher than the weight of lowly soluble saccharide.

(1.1.2 Other water-soluble saccharides)

The tablet of the present invention can contain water-soluble saccharides other than the above-mentioned highly soluble saccharide and lowly soluble saccharide. A water-soluble saccharide other than those having the highest solubility in water at 37°C (highly soluble saccharide) and those having the lowest solubility in water at 37°C (lowly soluble saccharide) among the water-soluble saccharides contained in the tablet for removing tongue coating of the present invention as the main components (preferably present at 10 % by weight or more, more preferably at 20 % by weight or more) is conveniently referred to as other water-soluble saccharides. The tablet of the present invention may contain, as water-soluble saccharides, only two types of water-soluble saccharides being highly soluble saccharide and lowly soluble saccharide, or may contain more types of water-soluble saccharides (e.g., a total of three, four, five, six, seven, eight, nine, ten, or more types of water-soluble saccharides).

The solubility of other water-soluble saccharides in water at 37°C is preferably higher than the solubility of lowly soluble saccharide by 5 % by weight or more, more preferably by 10 % by weight or more, and even more preferably by 15 % by weight or more.

The tablet of the present invention can contain, for example, sorbitol as the highly soluble saccharide, palatinit as the lowly soluble saccharide, and maltitol as the other water-soluble sugar alcohol.

In the tablet of the present invention, other water-soluble saccharides may be used in any amount within a weight range which can be covered by components other than the highly soluble saccharide and the lowly soluble saccharide. The total weight of the other water-soluble saccharides is preferably less than the total weight of the highly soluble saccharide and the weight of the lowly soluble saccharide. The weight of the other water-soluble saccharides which can be used in the tablet of the present invention can be, for example, about 60 % by weight or less, about 55 % by weight or less, about 50 % by weight or less, about 45 % by weight or less, about 40 % by weight or less, about 35 % by weight or less, about 30 % by weight or less, about 25 % by weight or less, about 20 % by weight or less, about 15 % by weight or less, about 10 % by weight or less, about 5 % by weight or less or the like, respectively.

(1.2 Other components)

The tablet of the present invention can optionally contain other components useful for removing tongue coating, as long as adverse effects regarding dissolution of water-soluble saccharides are not present. Examples of other components useful for removing tongue coating include compounds having an action of removing tongue coating, enzymes having an effect of improving the action of removing tongue coating, bacteriostatic agents, and pH adjusting agents.

Compounds having an action of removing tongue coating have an action of removing tongue coating. Compounds having an effect of removing tongue coating are preferably enzymes having an effect of removing tongue coating and are preferably protease, amylase, or lipase, more preferably protease, further preferably cysteine protease, and further preferably cysteine protease of the papain family. The enzyme is preferably bromelain, papain, or actinidine, and more preferably bromelain or papain.

Enzymes having effects to improve the action of removing tongue coating have effects to improve the action of removing tongue coating. Such enzymes are preferably selected from the group consisting of α-amylase, β-amylase, dextranase, trypsin, papain, protease, bromelain, pectinase, pepsin, peptidase, phospholipase, muramidase, lysozyme, and lipase, more preferably protease, more preferably cysteine protease, and more preferably cysteine protease of the papain family. This enzyme is preferably bromelain, papain, or actinidine, and more preferably bromelain or papain.

When the tablet of the present invention contains bromelain, the amount of bromelain is preferably about 0. 1 % by weight or more, more preferably about 0.2 % by weight or more, and most preferably about 0.5 % by weight or more. The amount of bromelain is preferably about 3 % by weight or less, more preferably about 2 % by weight or less, further preferably about 1. 5 % by weight or less, and most preferably about 1.0 % by weight or less. These amounts are particularly preferable when the tablet is dissolved in the oral cavity for over about 5 minutes to about 10 minutes. When the tablet is dissolved more quickly, the amount of bromelain is preferably less than that. When the tablet is dissolved more slowly, the amount of bromelain is preferably more than that.

When the tablet of the present invention contains papain, the amount of papain is preferably about 0.15 % by weight or more, more preferably about 0.3 % by weight or more, and most preferably about 0.75 % by weight or more. The amount of papain is preferably about 4.5 % by weight or less, more preferably about 3 % by weight or less, further preferably about 2.25 % by weight or less, and most preferably about 1.5 % by weight or less. These amounts are particularly preferable when the tablet is dissolved in the oral cavity for over about 5 minutes to about 10 minutes. When the tablet is dissolved more quickly, the amount of papain is preferably less than that. When the tablet is dissolved more slowly, the amount of papain is preferably more than that.

When the tablet of the present invention contains actinidine, the amount of actinidine is preferably about 0.35 % by weight or more, more preferably about 0.7 % by weight or more, and most preferably about 1.75 % by weight or more. The amount of actinidine is preferably about 10. 5 % by weight or less, more preferably about 7 % by weight or less, further preferably about 5.25 % by weight or less, and most preferably about 3.5 % by weight or less. These amounts are particularly preferable when the tablet is dissolved in the oral cavity for over about 5 minutes to about 10 minutes. When the tablet is dissolvedmore quickly, the amount of actinidine is preferably less than that. When the tablet is dissolved more slowly, the amount of actinidine is preferably more than that.

The "Bacteriostatic agent" refers to a substance that suppresses or inhibits bacterial proliferation. Particularly, the bacteriostatic agent preferably suppresses or inhibits proliferation of streptococci or staphylococci. An example of a bacteriostatic agent includes polyphenols (e.g., catechin). The bacteriostatic agent is preferably catechin.

Examples of the pH adjusting agents include, acids such as citric acid, phosphoric acid, carbonic acid, malic acid, acetic acid and the like, as well as salts thereof. The pH adjusting agent is preferably citric acid.

The tablet of the present invention can optionally contain additives which are normally contained in tablets, as long as adverse effects regarding dissolution of water-soluble saccharides are not present. Examples of additives include binders, flavors, sweeteners, coloring agent, excipients, disintegrants, lubricants, fluidizers, coating agents, and the like. These additives are well-known in the art. When the tablet is intended for food application, such additives are listed in, for example, Japanese Standards of Food Additives. When the tablet is intended for pharmaceutical application, such additives are listed in, for example, Japanese Pharmacopoeia.

As used herein, the term "binder" refers to an additive that gives binding force to mixtures of component powders and is used for production of stable tablets or granules. Although the tablet of the present invention does not necessarily need to contain the binder, the binder may be added when hardness of the tablet is insufficient.

Binders are preferably cellulose, gum arabic, gelatin, or the like.

Binders improve hardness of the tablet by binding substances included in the tablet. When a substance alone is compressed to obtain a tablet, the hardness of the tablet shows the level of the ability of the substance as a binder. The higher the hardness, the stronger the binding force.

(2. Production method of the tablet of the present invention)

The tablet of the present invention can be manufactured using the same method and facilities as those used for production of conventional tablets without adding any modification thereto. For example, it is possible to employ a method in which tablet materials (e.g., water-soluble saccharides and another component) are mixed and then are directly compressed into a tablet; a method in which granules of tablet materials are formed in a wet or dry state separately or granules of an admixture of the tablet materials are formed in a wet or dry state and the granules are compressed into tablets; or the like.

In one embodiment, the tablet of the present invention is preferably produced by mixing tablet materials in powder state and directly compressing them into a tablet.

In another embodiment, the tablet of the present invention is preferably manufactured by mixing tablet materials in the form of granules and compressing them into a tablet. In particular, it is preferable that granules of highly soluble saccharide and granules of lowly soluble saccharide are formed separately and then the granules are compressed into tablets. If granules of the highly soluble saccharide and granules of the lowly soluble saccharide are formed separately, concavities and convexities formed by sucking the tablet of the present invention become larger. Although the size of the granules of the highly soluble saccharide is arbitrary, the diameter thereof is preferably about 50 $\mu$m or more, more preferably about 75 $\mu$m or more, and most preferably about 90 $\mu$m or more. The granules of the highly soluble saccharide are preferably about 500 $\mu$m or less, more preferably about 350 $\mu$m or less, and most preferably about 250 $\mu$m or less. Although the size of the granules of the lowly soluble saccharide is arbitrary, a size thereof is preferably about 50 $\mu$m or more , more preferably about 75 $\mu$m or more, and most preferably about 90 $\mu$m or more. The particle size of the granules of the lowly soluble saccharide is preferably about 500 $\mu$m or less, more preferably about 350 $\mu$m or less, and most preferably about 250 $\mu$m or less. If granules having an appropriate size are used, concavities and convexities of an appropriate size are formed when sucking the tablet in the oral cavity.

(3. Tablet of the present invention)

The tablet of the present invention manufactured as mentioned above contains highly soluble saccharide and lowly soluble saccharide, the highly soluble saccharide and the lowly soluble saccharide are water-soluble, the highly soluble saccharide is a saccharide having the highest solubility in water at 37°C among the water-soluble saccharides contained in the tablet as the main components, the lowly soluble saccharide is a saccharide having the lowest solubility in water at 37°C among the water-soluble saccharides contained in the tablet as the main components, and the difference between the solubility of the highly soluble saccharide in water at 37°C and the solubility of the lowly soluble saccharide in water at 37°C is 10 % by weight or more

In the tablet of the present invention, highly soluble saccharide, lowly soluble saccharide, and other components may be dispersed uniformly, or may be localized.

Disintegration time of the tablet of the present invention is measured according to the Disintegration test method described in Japanese Pharmacopoeia. The disintegration time is preferably about 5 minutes or more, more preferably about 6 minutes or more, further preferably about 7 minutes or more, even more preferably about 8 minutes or more, particularly preferably about 9 minutes or more, and most preferably about 10 minutes or more. If the disintegration time is too short, there are some cases where an effect of removing tongue coating is not sufficiently exerted.

Although there is no particular upper limit for disintegration time of the tablet of the present invention, the disintegration time is preferably about 30 minutes or less, more preferably about 25 minutes or less, particularly preferably about 20 minutes or less, and most preferably about 15 minutes or less. This is because if the disintegration time is too long, the method may not be simple.

Hardness of the tablet of the present invention is measured using a Kiya-type hardness tester by loading the tablet in the direction of the diameter of the tablet, measuring the load resulting in destruction in respect to 5 tablets of each sample, and calculating their average. The average thus obtained is preferably about 15 kgf or more, more preferably about 20 kgf or more, and most preferably about 25 kgf or more. If hardness of the tablet is too low, there are some cases where disintegration in the oral cavity occurs too early and a sufficient effect of removing tongue coating is not obtained. Although there is no upper limit for the hardness of the tablet, for example, hardness up to 60 kgf, up to 50 kgf, or up to 40 kgf can be preferably selected.

Weight of the tablet of the present invention is preferably from about 0.05 g to about 10 g, more preferably from about 0.1 g to about 5 g, and further preferably from about 0.2 g to about 3 g. Only one tablet of the present invention may be sucked in the mouth, or a plurality of tablets (e.g. , 2 tablets to 10 tablets) may be sucked simultaneously. When a plurality of tablets are sucked simultaneously, a plurality of tablets may be put into the mouth at once, or may be put into the mouth sequentially one by one.

The tablet of the present invention can be of any shape. For example, the tablet may be in a disk shape, spherical shape, rugby ball shape, heart shape, or the like.

The tablet of the present invention has an action of removing tongue coating. The phrase "have an action of removing tongue coating" means that an action of removing tongue coating is confirmed by the following procedures. Specifically, a photograph of the dorsum of the tongue of a subject is taken according to the method shown in Example 2, the area of the dorsum of the tongue and the area of tongue coating are measured, and the tongue coating adhesion ratio is calculated based on this. Subsequently, in lieu of the tablet of Example 2, a tablet whose action of removing tongue coating should be confirmed (preferably about 1 tablet to about 10 tablets, more preferably about 1 tablet to about 5 tablets, and more preferably about 3 tablets) is given to the subject. Then the subject sucks it in a similar manner to that in Example 2 without biting, crushing, or swallowing. Normally, a tablet having the same size and same material as those of Example 2 takes about 5 to 15 minutes (e.g., about 10 minutes) for finishing sucking. Immediately after finishing sucking, the dorsum of the tongue is photographed, the area of the dorsum of the tongue and the area of tongue coating are measured, and the tongue coating adhesion ratio is calculated based on the areas. When the tongue coating adhesion ratio after taking a tablet is lower than the tongue coating adhesion ratio before taking the tablet, this tablet is referred to as a tablet having an action of removing tongue coating.

Tongue coating adhesion ratio is, as explained in Example 2, a ratio of the area of tongue coating-adhered portions to the area of the dorsum of the tongue. The area of tongue coating-adhered portions and the area of the dorsum of the tongue are obtained by taking a photograph of the dorsum of the tongue and by measuring each area. An example of area measurement method includes, for example, a method using computer software capable of executing image analysis. A computer software program capable of executing image analysis includes Photoshop Elements 2.0 made by Adobe System. When Photoshop Elements 2.0 by Adobe System is used, the contour of the dorsum of the tongue being photographed is manually scanned, the area inside the contour is counted as a number of pixels to measure the area of the dorsum of the tongue. At photographing, a color chart for color tone correction (CasMatch made by Dai Nippon Printing Co.,Ltd.) is placed alongside a photographic subject, and the brightness of the photograph is corrected using this. The area of tongue coating-adhered portions is measured by counting the number of pixels whose brightness is 150 or more inside the contour of the dorsum of the tongue.

Preferably, the number of pixels of tongue coating adhesion after taking the tablet is, as compared to the number of pixels of tongue coating adhesion before taking the tablet, lower by about 1% or more, more preferably lower by about 2% or more, further preferably lower by about 3% or more, further more preferably lower by about 4% or more, further more preferably lower by about 5% or more, further more preferably lower by about 10% or more, and further more preferably lower by about 15% or more. For example, if the tongue coating adhesion ratio before taking the tablet is 60%, the tongue coating adhesion ratio after taking the tablet is preferably about 59.4% or less, more preferably about 58.8% or less, further preferably about 58.2% or less, further preferably about 57.6% or less, further preferably about 57.0% or less, further preferably about 54.0% or less, and further preferably about 51.0% or less.

An action of removing tongue coating of the tablet of the present invention can be evaluated through evaluation of tongue coating scores. The tongue coating score is, as explained in Example 2, a total of the amount of tongue coating at sites [1] to [4] of the dorsum of the tongue when the amounts of tongue coating adhered to each of the dorsum of the tongue sites [1] to [4] in judgmental rating reference diagram for the amount of tongue coating shown in FIG. 2 are evaluated to each of sites of the dorsum of the tongue [1] to [4] as (Tongue coating area score) x (Tongue coating thickness score). The maximum tongue coating score is 36 and the minimum tongue coating score is 0. Although a lower tongue coating score is preferable, a state where no tongue coating is present is not preferable (no tongue coating at any of 4 evaluation sites , or a total of tongue coating scores at four locations from 0 to 3 points). It is noted that the normal range free from halitosis is preferably from 4 to 8 points (1 to 2 points for each of the four evaluation sites), most preferably 4 points (1 point for each of the four evaluation sites). Judgment criteria of the amount of tongue coating at each site are as follows:

(Table 2)

Criteria of scores of tongue coating adhesion area

0: No tongue coating

1: Ratio of tongue coating adhesion area is 1/3 or less

2: Ratio of tongue coating adhesion area is greater than 1/3 and less than 2/3

3: Ratio of tongue coating adhesion area is 2/3 or more

(Table 3)

Criteria of scores of tongue coating thickness

0: No tongue coating

1: Lingual papilla are observed

2: Lingual papilla are almost hidden

3: Lingual papilla are hidden

Preferably, tongue coating score after taking the

tablet is, as compared to tongue coating score before taking the tablet, about 1 point or more lower, more preferably about 2 points or more lower, further preferably about 3 points or more lower, even further preferably about 4 points or more lower, even further preferably about 5 points or more lower, even further preferably about 10 points or more lower, and even further preferably about 15 points or more lower.

When the tablet of the present invention is sucked in the oral cavity, highly soluble saccharide is dissolved quickly, while lowly soluble saccharide is dissolved slowly, and thus concavities and convexities are formed on the surface of the tablet. Contact of these concavities and convexities with tongue coating allows physical scraping of the tongue coating and removal thereof. It is considered that the greater the concavities and convexities, the greater the physically removing effects that are obtained. Ease of formation of concavities and convexities on the surface of the tablet can be confirmed by, for example, the following method. First, the tablet is put into a beaker containing 100 cc of water (although the temperature of it is arbitrary, the temperature is preferably about 37°C). The beaker is shaken gently (e.g., under a condition of shaking width of 20 mm, 60 rpm using a rotary shaker) for a predetermined time (e.g., about 3 minutes, about 5 minutes, about 7 minutes or the like). Thereafter, the tablet is taken out from the beaker and moisture is removed, then the tablet is observed under a laser microscope at appropriate magnification. The degree of the relief can be determined by measuring a difference between peaks and troughs of relief at several locations (e.g., 3 locations) and calculating the averages thereof. The difference between peaks and troughs of the relief is also referred to as the size of the relief. The size of the relief is preferably about 30 $\mu$m or more, more preferably about 50 $\mu$m or more, and further preferably about 70 $\mu$m or more. The degree of the relief of concavities and convexities can also be represented by surface roughness. Surface roughness is preferably expressed by the arithmetic average of roughness ($R_a$) defined by B0601-1994 in JIS (Japanese Industrial Standards). $R_a$ is determined as follows: a reference length is extracted from the roughness curve in the direction of the average line, an X-axis is defined in the direction of the average line of this extracted portion and a Y-axis is defined in the direction of longitudinal magnification. The roughness curvature is expressed by y = f (x). A value, which is obtained by the following equation and is expressed in micrometers ($\mu$m), is $R_a$.

[Equation 1]

$$R_a = \frac{1}{l} \int_0^l |f(x)| \, dx$$

wherein, $l$:reference length.

A cut-off value for obtaining $R_a$ is generally selected from the following six figures: 0.08, 0.25, 0.8, 2.5, 8, and 25 (Unit: mm).

Standard cut-off value: Standard cut-off values and standard values of evaluation length corresponding to the range of $R_a$ for obtaining $R_a$ are generally in accordance with divisions shown in Table A below.

(Table A)

| Table A Standard cut-off values and evaluation length for obtaining $R_a$ | | | |
|---|---|---|---|
| Range of $R_a$ ($\mu$m) | | Cut-off value $\lambda_c$ (mm) | Evaluation length $l_n$ (mm) |
| More than | Or less than | | |
| (0.006) | 0.02 | 0.08 | 0.4 |
| 0.02 | 0.1 | 0.25 | 1.25 |
| 0.1 | 2.0 | 0.8 | 4 |
| 2.0 | 10.0 | 2.5 | 12.5 |
| 10.0 | 80.0 | 8 | 40 |

The number in parentheses is a value for reference.

$R_a$ is a value reflecting fine concavity and convexity on the surface of the tablet. When the $R_a$ is greater, the concavities and convexities are larger and the effect of removing tongue coating is higher. When, in the tablet solubility test, $R_a$ at 3 minutes after starting immersion is 15 $\mu$m or more and $R_a$ at 7 minutes after starting immersion is 20 $\mu$m or more, an action of removing tongue coating is obtained. Since it is said that the lower limit of size of particles sensible by human tongue is 20 $\mu$m, if $R_a$ at 7 minutes after starting immersion is 20 $\mu$m or more, the tongue can sufficiently detect concavities and convexities. $R_a$ can be measured by a device known in the art. For example, $R_a$ can be measured

by a laser microscope (VK-8500 made by Keyence Corporation).

In the tablet solubility test, $R_a$ at 3 minutes after starting immersion is preferably about 15 $\mu$m or more, more preferably about 20 $\mu$m or more, and most preferably about 30 $\mu$m or more. In the tablet solubility test, $R_a$ at 3 minutes after starting immersion has no particular upper limit and can be, for example, about 100 $\mu$m or less, about 90 $\mu$m or less, about 80 $\mu$m or less, about 70 $\mu$m or less, about 60 $\mu$m or less or the like. In the tablet solubility test, $R_a$ at 7 minutes after starting immersion is preferably about 20 $\mu$m or more, more preferably about 25 $\mu$m or more, more preferably about 30 $\mu$m or more, more preferably about 40 $\mu$m or more, and most preferably about 50 $\mu$m or more. In the tablet solubility test, $R_a$ at 7 minutes after starting immersion has no particular upper limit and can be, for example, about 200 $\mu$m or less, about 150 $\mu$m or less, about 140 $\mu$m or less, about 130 $\mu$m or less, about 120 $\mu$m or less, about 110 $\mu$m or less or the like.

The tablet of the present invention can be used for any applications which need tongue coating removal. For example, the tablet of the present invention is preferably used for humans with tongue coating score of point 10 or more. The tablet for removing tongue coating of the present invention can be used for, for example, patients with cerebral stroke. Since it is known that in patients with cerebral stroke, pneumonia is induced by excessive accumulation of tongue coating, the tablet for removing tongue coating of the present invention can be used for prevention of pneumonia. Since tongue coating is known to be a primary cause for halitosis, the tablet for removing tongue coating of the present invention can be used for removal or prevention of halitosis.

As used herein, having "halitosis" means that exhaled breath has an unpleasant odor. In particular, having halitosis means that causative substances responsible for an unpleasant odor are present in the exhaled breath at the threshold or more. In a more particular case, having halitosis means that volatile sulfur compounds are present in the exhaled breath in an amount of the threshold or more. In a more particular case, volatile sulfur compound is selected from the group consisting of hydrogen sulfide, methyl mercaptan, and dimethyl sulfide. The threshold at which bad odor is detected when volatile sulfur compound is present in exhaled breath is 1. 5 ng/10 ml of exhaled breath for hydrogen sulfide, 0.5 ng/10 ml of exhaled breath for methyl mercaptan, and 0.2 ng/10 ml of exhaled breath for dimethyl sulfide.

When halitosis removing treatment is performed to a subject having halitosis, preferably, the treatment is performed so that the amount of at least one of the volatile sulfur compounds in exhaled breath after taking the tablet is less than the threshold for detecting bad odor, that is, hydrogen sulfide is less than 1.5 ng/10 ml of exhaled breath, methyl mercaptan is less 0.5 ng/10 ml of exhaled breath, or dimethyl sulfide is less than 0.2 ng/10 ml of exhaled breath. More preferably, treatment is performed so that all amounts of hydrogen sulfide, methyl mercaptan, and dimethyl sulfide are less than the threshold for feeling bad odor.

Preferably, the amount of any volatile sulfur compounds in the exhaled breath after taking the tablet is lower than the amount of the volatile sulfur compound in the exhaled breath before taking the tablet by about 5% or more, more preferably lower by about 10% or more, more preferably lower by about 15% or more, further more preferably lower by about 20% or more, further more preferably lower by about 25% or more, further more preferably lower by about 30% or more, further more preferably lower by about 45% or more, and further more preferably lower by about 50% or more.

Although detailed mechanisms are not understood, a tablet having an action of removing tongue coating has preferably an action of reducing production of volatile sulfur compounds by oral bacteria. Therefore, when the subject takes this tablet, generation of halitosis after uptake can be prevented for a certain period in addition to removing halitosis already present at the time of uptake. Accordingly, the tablet of the present invention can be used for both of removal and prevention of halitosis.

The tablet of the present invention can be used for any applications which require halitosis removal or prevention. For example, the tablet of the present invention is preferably used for humans with volatile sulfur compounds at the threshold (that is, 1.5 ng/10 ml of exhaled breath for hydrogen sulfide, 0.5 ng/10 ml of exhaled breath for methyl mercaptan, or 0.2 ng/10 ml of exhaled breath for dimethyl sulfide) or more. It is considered that the tablet of the present invention reduces production of volatile sulfur compounds due to oral bacteria by acting primarily on tongue coating.

(4. Method of using the tablet of the present invention)

The amount of uptake, uptake frequency, and uptake period of the tablet of the present invention are determined depending on the conditions of the subject, tongue coating scores of the subject, and the like. The amount of uptake of the tablet of the present invention is preferably about 0.1 g or more, more preferably about 0.2 g or more, further more preferably about 0.5 g or more, and further more preferably about 1 g or more per uptake. Although the amount of uptake of the tablet of the present invention has no particular upper limit, it is, for example, about 1000 g or less, about 750 g or less, about 500 g or less, about 250 g or less, about 100 g or less, about 50 g or less, about 40 g or less, about 30 g or less, about 20 g or less, about 10 g or less, about 7.5 g or less, about 5 g or less, about 4 g or less, about 3 g or less, about 2 g or less, about 1 g or less or the like per uptake.

The uptake frequency of the tablet of the present invention can be set arbitrarily. For example, the uptake frequency can be once or more a week, twice or more a week, three or more times a week, four or more times a week, five or more times a week, six or more times a week, seven or more times a week, once or more a day, twice or more a day, three or more times a day. Uptake frequency of the tablet of the present invention has no upper limit, and can be, for example, three times or less a day, twice or less a day, once or less a day, seven or less times a week, six or less times a week, five or less times a week, four or less times a week, three or less times a week, twice or less a week, and once or less a week.

Although the timing of uptake of the tablet of the present invention may be before a meal, after a meal or between meals, after a meal is preferable. Before a meal refers to a period from immediately before a meal to about 30 minutes before a meal, after a meal refers to from immediately after a meal to about 30 minutes after a meal, and between meals refers to from more than about 2 hours after a meal to more than about 2 hours before a subsequent meal.

The uptake period of the tablet of the present invention can be determined arbitrarily. The tablet of the present invention can be taken preferably for about 1 day or more, more preferably for about 3 days or more, and most preferably for about 5 days ormore. The tablet of the present invention can be taken preferably for about 1 month or less, more preferably for about 2 weeks or less, and most preferably for 10 days or less. When necessary, the tablet of the present invention may be taken substantially permanently.

At the time of uptake, the tablet of the present invention is preferably retained in the oral cavity without swallowing. The time for retention of the tablet of the present invention in the oral cavity is preferably for about 10 seconds or more, more preferably for about 1 minute or more, and further preferably for about 3 minutes or more. The time for retention of the tablet of the present invention in the oral cavity is preferably for about 30 minutes or less, more preferably for about 20 minutes or less, and further preferably for about 10 minutes or less. When retention time is too short, it is difficult to attain tongue coating removal effects.

The tablet of the present invention is preferably sucked without biting till the end.

With the tablet of the present invention, an effect of improving taste sensitivity is expected in addition to an effect of improving the hygienic status of the oral cavity due to removal of tongue coating. These effects are due to the fact that foods easily contact with lingual papilla after the removal of tongue coating which covers the surface of the tongue.

Examples

(Example 1 and Comparative Examples 1 to 4: Production of tablet using various water-soluble saccharides and solubility tests)

(A. Production)

The components were mixed so as to obtain compositions shown in Table 4 below. The mixtures were compressed into tablets under conditions of suction depth of 10 mm, compression pressure of 3 tons using a circular compression mold with a diameter of 13 mm. Each of the obtained tablets has a weight of 1.0 g. The diameter was 13 mm. The thickness was 6.7 mm.

(Table 4)

|  | Example 1 | Comparative Example 1 | Comparative Example 2 | Comparative Example 3 | Comparative Example 4 |
|---|---|---|---|---|---|
| Glucose spray dried |  | 98 parts |  |  |  |
| Maltitol | 26 parts |  | 98 parts |  |  |
| Sorbitol | 40 parts |  |  | 98 parts |  |
| Palatinit | 32 parts |  |  |  | 98 parts |
| Sugar ester B-370F | 2 parts | 2 parts | 2 parts | 2 parts | 2 parts |
| Total | 100 parts | 100 parts | 100 parts | 100 parts | 100 parts |

(B. Solution test)

Two tablets of Example 1 and Comparative Examples 1 through 4 were respectively put into a beaker containing 100 cc of water. The beakers were shaken using a rotary shaker under a condition of shaking width of 20 mm, 60 rpm. The temperature of the water was about 37°C. The tablets were taken out 3 minutes and 7 minutes after starting immersion. Moisture was removed. Thereafter, the surface of the tablets was observed with the naked eye. The surface was observed with a magnified image by a laser microscope (VK-8500 made by Keyence Corporation). Three arbitrary points on the surface of each of the tablet were observed under the laser microscope. The shape of the surface of the tablet was observed. Further, the size of the reliefs was measured using a profile measurement mode.

With the tablet based on a plurality of saccharides produced in Example 1, concavities and convexities were observed from 3 minutes after starting immersion, and many rough portions were observed on the surface. Changes in shape due to dissolution were less and it was found that concavities and convexities were persistent for a long-time.

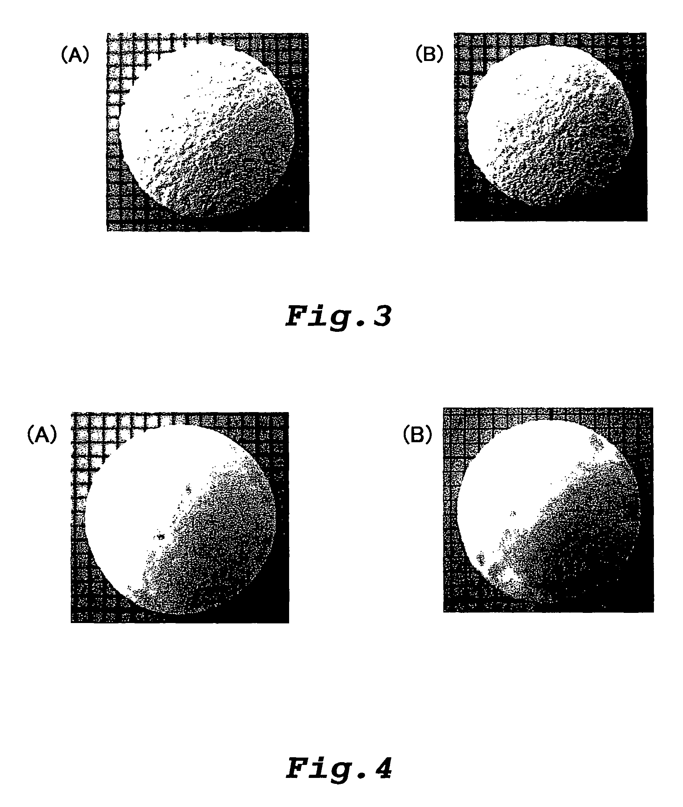

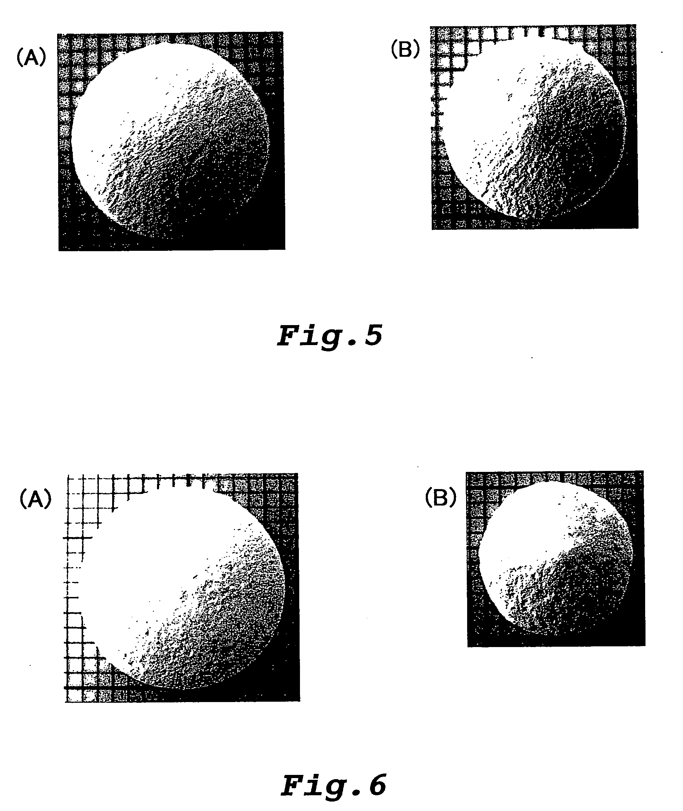

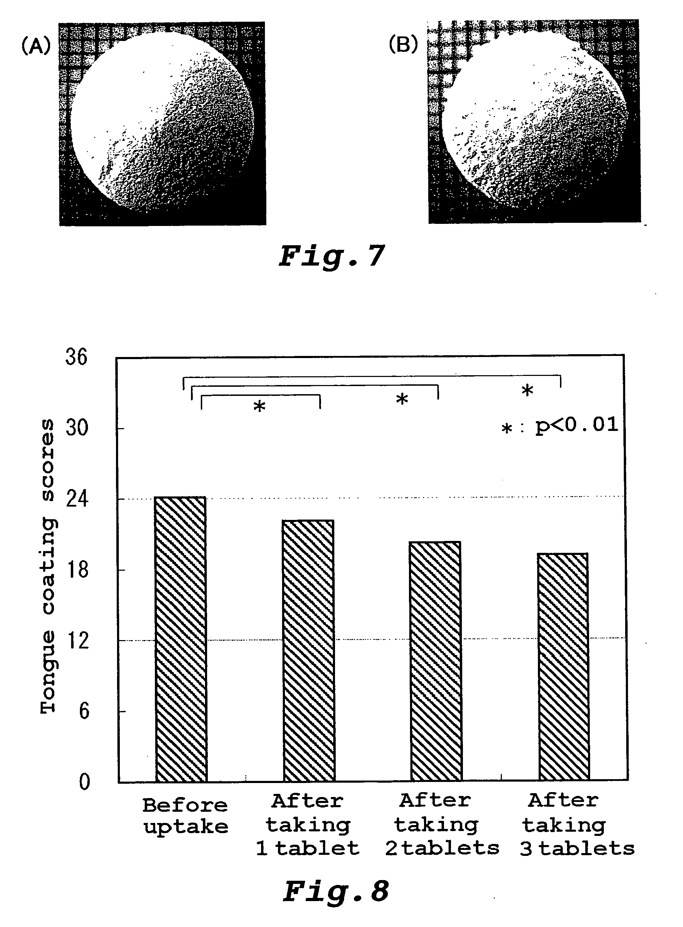

FIG. 3 (A) shows a photograph of the surface of the tablet of Example 1 taken at 3 minutes after immersion and FIG. 3 (B) shows a photograph of the surface of the tablet of Example 1 taken at 7 minutes after immersion. FIG. 4 (A) shows a photograph of the surface of the tablet of Comparative Example 1 taken at 3 minutes after immersion and FIG. 4 (B) shows a photograph of the surface of the tablet of Comparative Example 1 taken at 7 minutes after immersion. FIG. 5 (A) shows a photograph of surface of the tablet of Comparative Example 2 taken at 3 minutes after immersion and FIG. 5 (B) shows a photograph of the surface of the tablet of Comparative Example 2 taken at 7 minutes after immersion. FIG. 6 (A) shows a photograph of the surface of the tablet of Comparative Example 3 taken at 3 minutes after immersion and FIG. 6 (B) shows a photograph of the surface of the tablet of Comparative Example 1 taken at 7 minutes after immersion. FIG. 7 (A) shows a photograph of the surface of the tablet of Comparative Example 4 taken at 3 minutes after immersion and FIG. 7 (B) shows a photograph of the surface of the tablet of Comparative Example 4 taken at 7 minutes after immersion.

As a result of the observation of the surface with the naked eye performed at 3 and 7 minutes after immersion, more rough portions of surface of the tablet of Example 1, which used a plurality of saccharide raw materials, were observed than those of the tablets of Comparative Examples 1 to 4, which used single saccharide raw material. It is therefore considered that the tablet of Example 1 has greater physical tongue coating removing effects than tablets of Comparative Examples 1 to 4. With the glucose based tablet of Comparative Example 1, there was a tendency that the surface of the tablet was dissolved smoothly and that it was dissolved from the edge. With the maltitol based tablet of Comparative Example 2, although slightly rough portions were observed on the surface, concavities and convexities thereof were less. With the sorbitol based tablet of Comparative Example 3, although slight rough portions on the surface were able to be observed at 7 minutes after immersion, it became smaller quickly, and the dissolution rate was high. With the palatinit based tablet of Comparative Example 4, dissolution was worst, changes of it as a solid matter were slow, and small concavities and convexities on the surface were observed at 7 minutes after immersion.

By laser microscope observation of the tablets performed at 3 and 7 minutes after immersion, greater reliefs were observed on the surface of the tablet of Example 1 which used a plurality of saccharide raw materials compared to those of the tablets of Comparative Examples 1 to 4 which used single saccharide raw material. Volcanic rock-like clear reliefs were observed with the tablet of Example 1 which used a plurality of saccharide raw materials and sizes thereof were around from 70 to 80 μm. With the glucose based tablet of Comparative Example 1, the surface of it was smooth and glossy, and many fine crinklings were observed, while their reliefs were around 10 μm. With the maltitol based tablet of Comparative Example 2, small but rocky reliefs were observed here and there in relatively gentle reliefs, and their sizes were around from 40 to 60 μm. With the sorbitol based tablet of Comparative Example 3, slightly large crinklings were mixed in many fine crinklings, and reliefs thereof were around 50 μm. With the palatinit based tablet of Comparative Example 4, slightly smooth rocky-like reliefs were observed, and sizes of reliefs thereof were around 60 μm. It is noted that in the tablets of Example 1 and Comparative Examples 1 to 4, results of observation of the surface of the tablets at 3 minutes after immersion and the results of those of 7 minutes after immersion were almost the same, respectively.

The ratio of solubility of each tablet was in the order of Comparative Example 3 (based on sorbitol), Comparative Example 1 (based on glucose), Comparative Example 2 (based on maltitol), Example 1 (based on a plurality of saccharides), Comparative Example 4 (based on palatinit). Since it is considered that physical tongue coating removal effects are more effective with longer time spent by the tablet in the oral cavity, favorable results were obtained with the tablet of Example 1 in this regard. Further, with the tablet of Example 1, concavities and convexities appeared as early as 3 minutes later, and it was considered that this is the most ideal for removal of tongue coating.

In general, when using a tablet, single saccharide raw material is often used, and a case where several types of saccharide raw materials are formulated is rare. Thus, it is considered that the tablet of this type has better tongue coating removal effects as compared to other commercially available tablets.

(Comparative Example 5: Production of tablet using insoluble substances and evaluation thereof)

Tablets were produced with a similar manner as observed in Example 1 except that calcium carbonate was used in lieu of maltitol, sorbitol, and palatinit used in Example 1. This tablet was given to five male volunteers aged 30 to 45 years old who agreed to participate in the study after full explanation about the contents of the study was given. They are instructed to suck the tablet under the same conditions as Example 2, and evaluations upon completion of their sucking were obtained.

From the viewpoint of changing solubility of the base of the tablet, addition of insoluble substances (e.g. , calcium carbonate, or the like) can attain the object. However, after sucking the tablet, bleeding was reported due to that tongue was scraped by the insoluble substances beyond necessity. Therefore, it is considered that a tablet to be dissolved by sucking should be composed of water-soluble substances only.

(Example 2: Production of the tablet and tests of an effect of removing tongue coating thereof)

(A. Production of tablet)

Raw materials were mixed so as to obtain the composition ratio shown in Table 5 below. The mixture was compressed into tablets under conditions of a suction depth of 10 mm, compression pressure of 3 tons using a circular compression mold with a diameter of 13 mm. Each of the obtained tablets has a weight of 1.0 g. The diameter was 13 mm. The thickness was 6.7 mm.

(Table 5)

| Formulation of raw materials for test tablet | |
| --- | --- |
| Name of raw material | Formulating ratio (%) |
| Palatinit | 60.0 |
| Maltitol | 36.6 |
| Aspartame | 0.3 |
| Sucrose fatty acid esters | 3.0 |
| Fine silicon dioxide | 0.1 |
| Total | 100.0 |

(B. Subjects of the test)

Eleven male volunteers aged 28 to 49 years old, who agreed to participate in the study after full explanation about the contents of the studywas given, wereusedas subjects. It is noted that since this test was to examine an effect of removing tongue coating by taking a tablet, presence or absence of tongue coating was confirmed by preliminary observation in advance, and those who had little tongue coating were excluded from being subjects of the study. The subjects were instructed to prohibit tongue cleaning from 3 days prior to the experiment till the day of the experiment, and to prohibit all oral cavity activities including oral cavity cleaning, drinking, eating, and smoking from waking up in the morning of the day of the experiment till completion of the experiment.

(C. Tablet uptake test)

Instructions were given to the subjects such that the subjects took total three test tablets one by one, and the amounts of adhesion of tongue coating before and after the uptake were evaluated. Instructions were given to the subjects such that the subjects took the tablets by sucking mainly on the dorsum of the tongue without biting so as to dissolve the tablets slowly. The tablets of the present invention were a sugarless type (saccharide content less than 0.5%) using palatinit and maltitol as the base material. Since the dissolution rates of palatinit and maltitol in the mouth are different, concavity and convexity are generated on the surface of the tablet while being dissolved by sucking. Further, since the tablets are large, it takes 5 to 10 minutes before completion of sucking.

A photograph of the dorsum of the tongue was taken using a digital camera immediately before uptake of a tablet, immediately after one tablet uptake, immediately after two tablets uptake, immediately after three tablets uptake. Photographs of the dorsum of the tongue thus taken were evaluated by the method described under " C.1 Method of evaluation of the amount of tongue coating."

(C.1 Method of evaluation of the amount of tongue coating)

(1) Method of taking photograph

Photographs of the dorsum of the tongue of the subjects were taken using a digital camera (FinePix S602, FUJIFILM) equipped with ring flash (14RDX, YUZO). At photographing, a color chart for color tone correction (CasMatch, Dai Nippon Printing Co. , Ltd.) is placed alongside a photographic subject, and the brightness of the photograph was corrected using this.

(2) Method of evaluation by visual inspection

Evaluation of the amount of tongue coating by visual inspection was performed according to the method by Moritani et al. [7)] with adding considerations for thickness of the tongue coating. That is, the dorsum of the tongue photographed was divided into four areas, the amount of tongue coating adhered in each area was evaluated as (Tongue coating adhesion area score) x (Tongue coating thickness score), and total of these scores was used as the tongue coating score. Each Tongue coating adhesion area score and tongue coating thickness score was defined to be one of four levels of 0 to 3.
**[0140]**

　　1) The dorsum of the tongue is divided into four areas;
　　2) The amounts of adhered tongue coating are evaluated from area of tongue coating adhered to each area and thickness of tongue coating of each area;
　　(Tongue coating adhesion area score) x (Tongue coating thickness score)
　　3) The total of scores of each area is used as the tongue coating score.

The criteria of scores of tongue coating adhesion area was as follows:
(Table 6)
Criteria of scores of tongue coating adhesion area
0: No tongue coating
1: Ratio of tongue coating adhesion area is 1/3 or less
2: Ratio of tongue coating adhesion area is greater than 1/3 and less than 2/3
3: Ratio of tongue coating adhesion area is 2/3 or more
The criteria of scores of tongue coating thickness was as follows:
(Table 7)
Criteria of scores of tongue coating thickness
0: No tongue coating
1: Lingual papilla are observed
2: Lingual papilla are almost hidden
3: Lingual papilla are hidden

(3) Method of evaluation by image analysis

Evaluation of the amount of tongue coating by image analysis was performed as follows using software Photoshop Elements 2.0 made by Adobe System. The contour of the dorsum of the tongue in a photograph was scanned and the total number of pixels within the contour was used as the area of the dorsum of the tongue. On the dorsum of the tongue, the total of number of pixels whose brightness is 150 or more was used as the tongue coating adhesion area. Tongue coating adhesion ratio was obtained by dividing tongue coating adhesion area by area of the dorsum of the tongue. Evaluation by image analysis was carried out for p9 subject only.

(4) Statistical analysis

Results obtained by visual inspection and method of evaluation by image analysis were evaluated by paired t-test before taking the tablet and immediately after taking 1 tablet to 3 tablets.

(C.2 Test results)

(C.2.1 Results of evaluation by visual inspection)

Results of each of 11 subjects are shown below. As a result, tongue coating was reduced in 7 subjects after taking 1 tablet as compared to before uptake. After taking 2 tablets, tongue coating was reduced as compared to before uptake in 8 subjects. After taking 3 tablets, tongue coating was reduced as compared to before uptake in 9 subjects.
(Table 8)

| Subject | Before uptake | After taking 1 tablet | After taking 2 tablets | After taking 3 tablets |
|---|---|---|---|---|
| p1 | 26 | 22 | 22 | 22 |
| p2 | 18 | 18 | 18 | 18 |
| p3 | 10 | 8 | 5 | 5 |
| p4 | 30 | 30 | 30 | 30 |
| p5 | 36 | 36 | 30 | 30 |
| p6 | 24 | 21 | 18 | 16 |
| p7 | 22 | 20 | 18 | 18 |
| p8 | 6 | 4 | 4 | 4 |
| p9 | 33 | 30 | 26 | 22 |
| p10 | 30 | 30 | 30 | 26 |
| p11 | 30 | 24 | 21 | 20 |
| Average | 24.09 | 22.09 | 20.18 | 19.18 |
| p-value | | 0.007 | 0.002 | 0.001 |

Tongue coating score was 24.1 ± 9.0 before taking the tablet. Tongue coating score was reduced depending on the amount of tablets taken as to 22.1 ± 9.2 after taking 1 tablet, to 20.2 ± 8.7 after taking 2 tablets, and to 19.2 ± 8.2 after taking 3 tablets. Tongue coating score after taking 1 tablet was reduced significantly from before uptake ($p < 0.01$). The results are shown in FIG. 8. There is a significant difference between the results before uptake and after taking 1 tablet, between the results before uptake and after taking 2 tablets, and between the results before uptake and after taking 3 tablets.

(C.2.2 Results of evaluation by image analysis)

Image analysis was performed for above-mentioned p9 subject. The results are as follows. As a result, tongue coating was either reduced after taking 1 tablet, after taking 2 tablets, and after taking 3 tablets as compared to before uptake, and at the same time, the amount of tongue coating was reduced every time after 1 tablet was taken.
(Table 9)

| Subject | Before uptake | After taking 1 tablet | After taking 2 tablets | After taking 3 tablets |
|---|---|---|---|---|
| p9 | 64.8 | 51.5 | 50.5 | 46.8 |

(C.3 Conclusions)

From the test results as shown above, it is confirmed that tongue coating adhesion amount is reduced by taking the tablet. It is considered that tongue coating removal by taking the tablet is efficacious as one of routine oral care means from the viewpoint of convenience of use and safety.
(Examples 3 to 6: Production of enzyme-free tablets and enzyme-containing tablets, and a test of an effect of removing tongue coating thereof)

(A. Production of tablet)

Raw materials were mixed so as to obtain the composition ratio shown in Table 10 below. The mixtures were compressed into tablets under conditions of suction depth of 10 mm, compression pressure of 3 tons using a circular compression mold with a diameter of 13 mm. Each of the obtained tablets has a weight of 1.0 g. The diameter was 13 mm. The thickness was 6.7 mm.

(Table 10)

| Formulation of raw materials for test tablet | | | | |
|---|---|---|---|---|
| Name of raw material | Example 3 Formulating ratio (%) | Example 4 Formulating ratio (%) | Example 5 Formulating ratio (%) | Example 6 Formulating ratio (%) |
| Bromelain | - | 0.5 | - | - |
| Papain | - | - | 0.35 | 0.23 |
| Palatinit | 64.8 | 64.3 | 64.45 | 64.57 |
| Maltitol | 33.0 | 33.0 | 33.0 | 33.0 |
| Aspartame | 0.1 | 0.1 | 0.1 | 0.1 |
| Sucrose fatty acid esters (HLB3) | 2.0 | 2.0 | 2.0 | 2.0 |
| Fine silicon dioxide | 0.1 | 0.1 | 0.1 | 0.1 |
| Total | 100.00 | 100.00 | 100.00 | 100.00 |

(B. Subjects of the test)

For any of Examples 3 to 6, nine male volunteers aged 32 to 54 years old, who agreed to participate in the study after full explanation about the contents of the study was given, were used as subjects. It is noted that since this test was to examine an effect of removing tongue coating by taking the tablet, presence or absence of tongue coating was confirmed by preliminary observation in advance, and those who has little tongue coating were excluded from being subjects of the study. The subjects were instructed to prohibit tongue cleaning from 3 days prior to the experiment till the day of the experiment, and to prohibit all oral cavity activities including oral cavity cleaning, drinking, eating, and smoking from waking up in the morning of the day of the experiment till completion of the experiment.

(C. Tablet uptake test)

Instructions were given to the subjects such that the subjects took a total of three test tablets one by one, and the amounts of adhesion of tongue coating before and after the uptake were evaluated. Instructions were given to the subjects such that the subjects took the tablets by sucking them mainly on the dorsum of the tongue without biting so as to dissolve the tablet slowly. The tablets of the present invention were a sugarless type (saccharide content less than 0.5%) using palatinit and maltitol as the base material. Since dissolution rates of palatinit and maltitol in the mouth are different, concavities and convexities are generated on the surface of the tablet while being dissolved by sucking. Further, since the tablets are large, it takes 5 to 10 minutes before completion of sucking. Time needed for completion of sucking 1 enzyme-free tablet (Example 3) was 6 minutes and 42 seconds on average. The period of the time needed for completion of sucking 1 papain containing tablet (Examples 5 and 6) was 6 minutes and 43 seconds on average.

A photograph of the dorsum of the tongue was taken using a digital camera immediately before uptake of the tablet, immediately after one tablet uptake, immediately after two tablets uptake, immediately after three tablets uptake. Photographs of the dorsum of the tongue thus taken were evaluated by the method described under (2) Method of evaluation by visual inspection in "C.1 Method of evaluation of the amount of tongue coating" of Example 2.

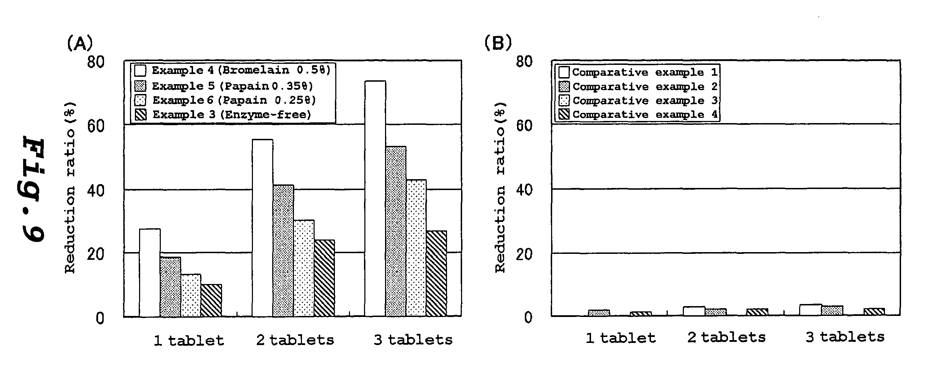

The results of the reduction ratios of the tongue coating score are shown in Table 11 below and FIG. 9 (A). Here, the reduction ratio of the tongue coating scores denotes the reduction percentages of the tongue coating score assuming that the tongue coating score before taking the tablet is 100%.

(Table 11)

| Reduction ratio (%) of tongue coating scores | | | | |
|---|---|---|---|---|
| | Enzyme | Reduction ratio (%) of tongue coating scores | | |
| | | After taking 1 tablet | After taking 2 tablets | After taking 3 tablets |
| Example 3 | No enzyme | 9.9 | 24.0 | 26.8 |
| Example 4 | 0.5% Bromelain | 27.6 | 55.4 | 73.6 |
| Example 5 | 0.35% Papain | 18.7 | 41.2 | 53.1 |
| Example 6 | 0.23% Papain | 13.4 | 30.3 | 42.8 |

As a result, it is found that uptake of the tablet of the present invention results in reduction in the tongue coating score. The tablet of the present invention, even if no enzyme is contained, has effects for removing tongue coating. When the tablet of the present invention contains enzyme, the effects for removing tongue coating was enhanced. As for the enzyme, both papain and bromelain were effective and in particular, effects by bromelain were high. Actions of the enzymes were approximately proportional to concentrations of the enzymes. Uptake of an enzyme-free tablet could reduce tongue coating by about 10% per tablet. A bromelain containing tablet reduced 35% of tongue coating per 1% of concentration of bromelain. A papain containing tablet caused 25% reduction of tongue coating per 1% of concentration of papain.

(Examples 7 and 8: Production of enzyme-free tablet and actinidine containing tablet, and tests of an effect of reducing volatile sulfur compounds thereof)

(A. Production of tablet)

Raw materials were mixed so as to obtain the composition ratios shown in Table 12 below. The mixtures were compressed into tablets under conditions of a suction depth of 10 mm, compression pressure of 3 tons using a circular compression mold with a diameter of 13 mm. Each of the obtained tablets has a weight of 1.0 g. The diameter was 13 mm. The thickness was 6.7 mm. Actinidine (EC 3.4.22.14) was obtained from Asahi Food and Health Care Co. , LTD. This actinidine was derived from cultivar Hayward of kiwi fruit, and had enzyme activity of 9000 unit/g (determined by a method in accordance with papain specified in Japanese Standards of Food Additives, Ver. 7 and casein was used as the substrate) was obtained.

(Table 12)

| Formulation of raw materials for test tablet | | |
|---|---|---|
| Name of raw material | Example 7 Formulating ratio (%) | Example 8 Formulating ratio (%) |
| Actinidine | - | 3.0 |
| Palatinit | 64.8 | 61.8 |
| Maltitol | 33.0 | 33.0 |
| Aspartame | 0.1 | 0.1 |
| Sucrose fatty acid esters (HLB3) | 2.0 | 2.0 |
| Fine silicon dioxide | 0.1 | 0.1 |
| Total | 100.00 | 100.00 |

(B. Subjects of the test)

The present Example was to examine effects of the tablet of the present invention against volatile sulfur compounds (hereafter abbreviated as VSC), and therefore, male adult volunteers comply with the following conditions (1) to (3) were used as subjects.

(1) Prior to the experiment, VSC at the threshold or more is detected in oral cavity air.

(2) In the results of intraoral inspection, there is 8 or more periodontal pockets with the depth of 4 mm or more, there is no periodontal pocket with a depth of 6 mm or more, and bleeding is at 10 locations or less.
(3) As the results of an X-ray inspection, no resorption of the alveolar bone is observed.

Fourteen volunteers aged 31 to 46 years old, who agreed to participate in the study after full explanation about the contents of the study was given, were instructed to prohibit tongue cleaning from 3 days prior to the experiment till the day of the experiment, and to prohibit all oral cavity activities including oral cavity cleaning, drinking, eating, and smoking from getting up in the morning of the day of the experiment till completion of the experiment.
It is noted that the tests were carried out based on approval by Ethical Examination Board of Ezaki Glico Co. , Ltd. in accordance with the spirit of the Helsinki Declaration.

(C. Tablet uptake test)

(C.1 Method of taking tablet)

Crossover tests in which all subjects were used as subject for both the tablet of Example 7 (enzyme-free) and the tablet of Example 8 (actinidine containing) were carried out. Eleven subjects are assigned at random either to a 6-membered group or a 5-membered group, in which one group first took the tablet of Example 7 and the other group first took the tablet of Example 8.
Instructions were given to the subjects such that the subjects took 1 test tablet. VSC concentrations in the oral cavity air were measured immediately before use of the tablet, immediately after use, 30 minutes later, 60 minutes later, and 90 minutes later. Instructions were given to the subjects such that the subjects took the tablet by sucking mainly on the dorsum of tongue without biting so as to dissolve the tablet slowly. The tablet of the present invention was a sugarless type (saccharide content less than 0.5%) using palatinit and maltitol as the base material. Since dissolution rates of palatinit and maltitol in the mouth are different, concavities and convexities are generated on the surface of the tablet while being dissolved by sucking. Further, since the tablet is large, it takes about 5 to 10 minutes before completion of sucking. Time needed to complete sucking 1 tablet was 6 minutes and 44 seconds on average of both the group with the enzyme-free tablet (Example 7) and the group with the enzyme-containing tablet (Example 8).
Subsequently, after at least a 2 week washout period, tablets to be used are switched and the same test was carried out. A double-blind method was employed for the test method in which the type of the tablet used was not told to subjects nor the measuring person. VSC concentrations in the oral cavity air were measured by the method described under "C.2 Method of halitosis evaluation".

(C.2 Method of halitosis evaluation)

As for VSC, attention was focused on $H_2S$ and $CH_3SH$, since the degree of contribution of $H_2S$ and $CH_3SH$ to halitosis is high. Concentrations of $H_2S$ and $CH_3SH$ were determined using a gas chromatograph equipped with flame photometric detector which uses an automatic sample injection device (Shimadzu Corporation, Kyoto). The subjects were instructed to breathe deeply once, and then to hold the tube connected to the automatic sample injection device in the mouth without leaking breath, and to breathe through the nose while resting for 60 seconds. After that, 25 ml of breath was collected and 10 ml of this was injected from the automatic sample injection device into the gas chromatograph. Concentrations of $H_2S$ and $CH_3SH$ were calculated by weight in 10 ml of oral cavity air (ng/10 ml) by the analysis software GC-Solution (Shimadzu Corporation, Kyoto) in a personal computer connected to the gas chromatograph. Throughout the tests, collection of the sample and measurement of concentrations of $H_2S$ and $CH_3SH$ were performed by the same measuring person starting at between 9:00 AM and 9:30 AM.

(C.3 Statistical analysis)

For the results obtained, t-tests were performed for relative concentrations of $H_2S$ and $CH_3SH$ after tablet use, considering the respective concentrations of $H_2S$ and $CH_3SH$ immediately before tablet use were 100%. The results were considered to be significantly different when $p < 0.05$ or less. For statistical processing, SPSS 10.0J for Windows (registered trade mark) (SPSS Inc., USA) was used.

(C.4 Results)

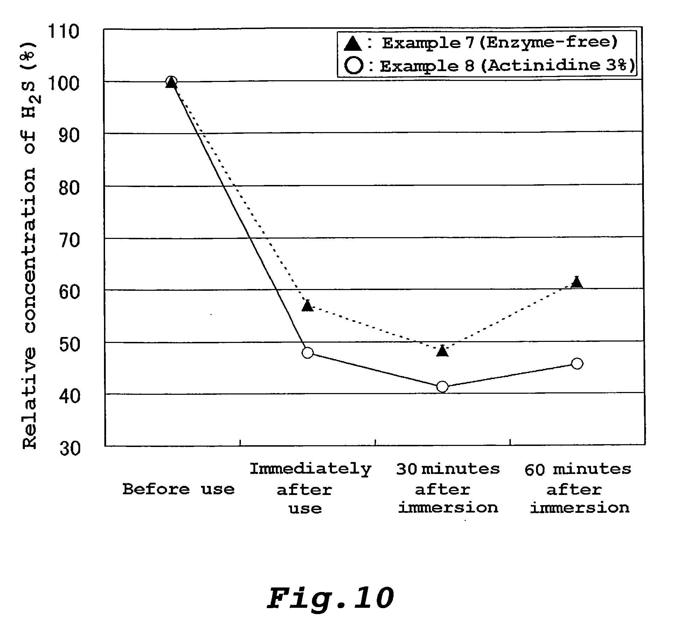

The results of relative changes (%) of $H_2S$ concentration are shown in Table 13 below and FIG. 10.
(Table 13)

| | Relative concentration (%) of $H_2S$ | | | |
|---|---|---|---|---|
| | Before use | Immediately after use | 30 minutes after use | 60 minutes after use |
| Example 7 (No enzyme) | 100 | 56.97 | 48.26 | 61.32 |
| Example 8 (actinidine 3%) | 100 | 47.89 | 41.30 | 45.63 |

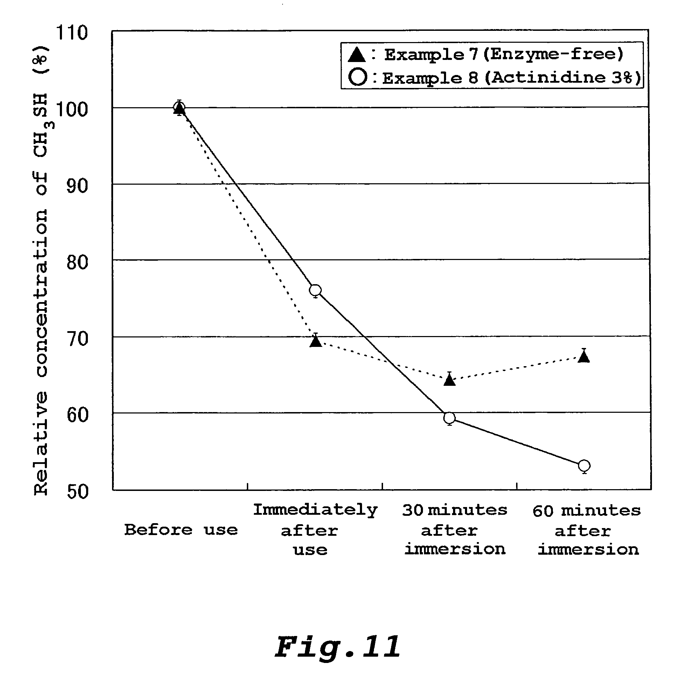

The results of relative changes (%) of $CH_3SH$ concentration are shown in Table 14 below and FIG. 11.

(Table 14)

| | Relative concentration (%) of $CH_3SH$ | | | |
|---|---|---|---|---|
| | Before use | Immediately after use | 30 minutes after use | 60 minutes after use |
| Example 7 (No enzyme) | 100 | 69.44 | 64.33 | 67.37 |
| Example 8 (actinidine 3%) | 100 | 76.04 | 59.37 | 53.11 |

Immediately before tablet use, $H_2S$ and $CH_3SH$ at the threshold or more were detected from all the subjects.

By using any of the tablets in Example 7 or Example 8, concentrations of $H_2S$ and $CH_3SH$ with respect to any of immediately after tablet use, 30 minutes after use, and 60 minutes after use were reduced as compared to those of immediately before tablet use. Particularly, when the actinidine containing tablet was used, effects of reducing $H_2S$ and $CH_3SH$ persisted for a longer time.

As a result of visual inspection of the dorsum of the tongue performed immediately before the experiment, adhesion of tongue coating was observed with all the subjects. As shown in Examples 2 to 6, it was revealed that tongue coating is reduced with the use of the tablet of the present invention and that much more tongue coating is removed by incorporating an enzyme into the tablet of the present invention. Therefore, regarding the reasons for reduction in $H_2S$ concentration and $CH_3SH$ concentration in both Examples 7 and 8 between immediately after the use of the tablet and 60 minutes after the use, it is considered as follows. Tongue coating that is a source generating $H_2S$ and $CH_3SH$ is removed or decomposed by the use of the tablet of the present invention. The saliva secretion was accelerated during the period of 6 minutes and 45 seconds, which is the average time of use of the tablet. Further, putrid saliva was washed out. From these results, it was suggested that the tablet of the present invention is an efficacious means for reduction of physiological halitosis and pathological halitosis. Particularly, it was suggested that the tablet of the present invention containing the enzyme is a highly effective means for reduction of physiological halitosis and pathological halitosis.

The chemical cleaning method of tongue coating using the tablet of the present invention is simpler and has lower risk for causing mechanical irritation in the oral cavity in comparison with mechanical tongue coating cleaning method using an instrument. Therefore, this method is considered to be an effective means to continue cleaning the tongue habitually. Further, simplicity in use and higher safety are considered to be especially useful when patients having insufficient physical functions such as elderly persons and diseased persons clean their tongue by themselves. The aforementioned Example suggests that the chemical cleaning method of tongue coating using the tablet of the present invention is an efficacious means for reduction of halitosis.

(Examples 9 and 10: Production of troches containing no enzyme and troches containing actinidine, and tests for their effects of removing tongue coating)

For the sake of development of a simple chemical cleaning method relating to removal of tongue coating of those who require nursing care, studies were made on changes in the amounts of tongue coating adhered through continued uptake of a troche containing a protease derived from kiwi fruit.

(A. Production of troches)

Raw materials were mixed so as to obtain the compositions shown in Table 15 below. The mixture was subjected to compression under conditions of compression pressure of 2 tons using a compression mold of a doughnut shape with a diameter of 19 mm and a center part of 8 mm, and troches were obtained. One tablet thus obtained has a weight of 1.5 g, has a diameter of 19 mm, a 8 mm hole at the center and a thickness of 4.5 mm. Actinidine (EC 3.4.22.14) is derived from Hayward species kiwi fruit and has an enzyme activity of 9000 unit/g (determined by using a method using casein as a substrate, in accordance with papain described in Japanese Standards of Food Additives, Ver. 7). The actinidine was obtained from Asahi Food and Health Care Co., LTD.

(Table 15)

| Formulation of the raw materials for the test tablets | | |
|---|---|---|
| Name of raw material | Example 9 mixing ratio (%) | Example 10 mixing ratio (%) |
| Actinidine | - | 3.0 |
| Palatinit | 64.8 | 61.8 |
| Maltitol | 33.0 | 33.0 |
| Aspartame | 0.1 | 0.1 |
| Sucrose fatty acid esters (HLB3) | 2.0 | 2.0 |
| Fine silicon dioxide | 0.1 | 0.1 |
| Total | 100.00 | 100.00 |

(B. Subjects of the test)

Among those who utilize special elderly nursing homes and have tongue coating adhesion, 8 persons (5 women, average age: 85.6 ± 7.6 years old) who agreed to participate in the study, were assigned to the study. Since this test was to investigate an effect of removing tongue coating by troche uptake, presence or absence of tongue coating was confirmed by preliminary observation in advance, and those who had little tongue coating were excluded from being subjects of the study.

(C. Troche uptake test)

Instructions were given to three subjects such that the three subjects took the troche described in Example 9, which contains no enzyme. Instructions were given to five subjects such that the five subjects took the actinidine-containing troche (enzyme-containing troche) described in Example 10. Uptake frequency of the troche was twice a day (after breakfast and after dinner) and at each point, one troche was given. Instructions were given to the subjects such that the subjects took the troches by sucking mainly on the dorsum of the tongue without biting so as to dissolve it slowly. The troche of the present invention is of sugarless type (saccharide content less than 0.5%) using palatinit and maltitol as the base materials. Since the dissolution rates of palatinit and maltitol in the mouth are different, concavities and convexities are formed on the surface of the troche while being dissolved by sucking. Further, since the troches are large, it takes about 5 to 10 minutes before completion of sucking.

Photographs of the dorsum of the tongue were taken during uptake of the troche, before taking the troche, after wake-up and before breakfast on day 3 and day 7. The amounts of tongue coating were evaluated with three persons who performed sufficient calibration in accordance with "(2) Method of evaluation by visual inspection" in "C.1 Method of evaluation of the amount of tongue coating" described in Example 2.

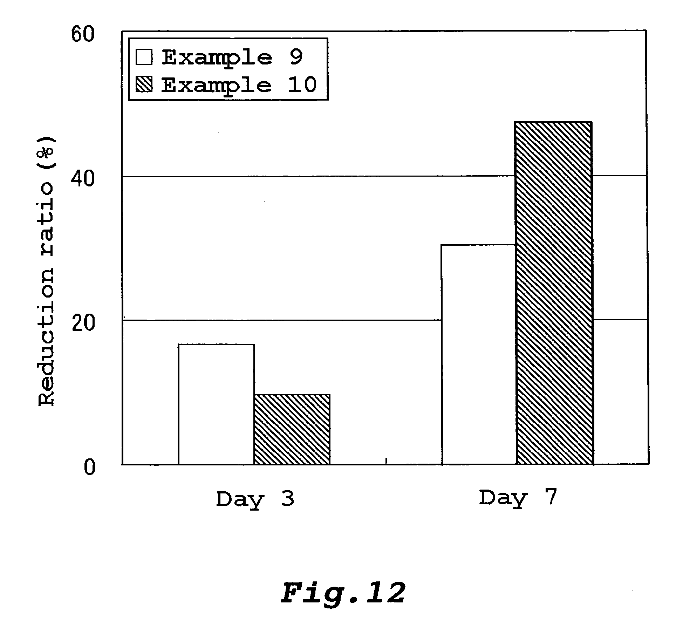

The results of the reduction ratio of the tongue coating score are shown in Table 16 and FIG. 12 below. Here, the reduction ratio of the tongue coating scores denotes the reduction percentages of the tongue coating score assuming that the tongue coating score before taking the tablets is 100%.

(Table 16)

| | Reduction ratio of tongue coating scores (average) | |
|---|---|---|
| | day 3 | day 7 |
| Example 9 (no enzyme) | 16.7 | 30.5 |
| Example 10 (actinidine 3%) | 9.8 | 47.3 |

With the results thus obtained, reduction in tongue coating scores was observed with the troches shown in both Example 9 and Example 10. The results suggested that the troche of the present invention is an efficacious means of a chemical cleaning method of tongue coating. It was also suggested that an enzyme-containing troche is particularly excellent as the troche of the present invention.

The chemical cleaning method using a troche has lower risk of causing mechanical irritation in the oral cavity and the method is simpler in comparison with a physical cleaning method using an instrument. Therefore, this method is considered to be effective when elderly persons, who require nursing care, are able to perform tongue cleaning by themselves.

(Comparative Examples 1 to 4: Effect of removing tongue coating of tablets using single saccharide)

Tongue coating removal effects of the tablet manufactured in Comparative Examples 1 to 4 were confirmed by the following procedures.

(B. Subjects of the test)

For each of Comparative Examples 1 to 4, five male volunteers from 32 to 54 years old of age, who agreed to participate in the study after full explanation about the details of the study was given, were assigned to the study. Since this test was to investigate the effect of removing tongue coating by taking tablets, presence or absence of tongue coating was confirmed by preliminary observation in advance, and those who have little tongue coating were excluded from being subjects of the study. Instructions were given to the volunteers to prohibit tongue cleaning from 3 days prior to the experiment till the day of the experiment, and to prohibit all oral cavity activities including oral cavity cleaning, drinking, eating, smoking and the like from rising in the morning of the day of the experiment till completion of the experiment.

(C. Tablet uptake test)

Instructions were given to the subjects such that the subjects took a total of three test tablets one by one, and the amounts of adhesion of tongue coating before and after the uptake were evaluated. Instructions were given to the subjects such that the subjects took the tablets by sucking them mainly on the dorsum of the tongue without biting so as to dissolve the tablets slowly. Since the tablets are large, it takes about 5 to 10 minutes before completion of sucking.

Photographs of the dorsum of the tongue was taken using a digital camera immediately before taking the tablet, immediately after taking the tablet , immediately after taking two tablets, immediately after taking three tablets. The photographs of the dorsum of the tongue thus taken were evaluated by the method described in "(2) Method of evaluation by visual inspection" in "C.1 Method of evaluation of the amount of tongue coating" described in Example 2.

The results of the reduction ratio of the tongue coating score are shown in Table 17 and FIG. 9 (B) below. Here, the reduction ratio of the tongue coating scores denotes the reduction percentages of the tongue coating score assuming that the tongue coating score before taking the tablets is 100%.

(Table 17)

| Reduction ratios of the tongue coating scores | | | | |
|---|---|---|---|---|
| | Types | Reduction ratios of the tongue coating scores (%) | | |
| | | After taking 1 tablet | After taking 2 tablets | After taking 3 tablets |
| Comparative Example 1 | Only glucose spray dried | 0.0 | 3.0 | 3.5 |
| Comparative Example 2 | Only maltitol | 2.1 | 2.3 | 3.2 |
| Comparative Example 3 | Only sorbitol | 0.0 | 0.0 | 0.0 |
| Comparative Example 4 | Only palatinit | 1.2 | 2.0 | 2.0 |

As a result, it is understood that regarding the tablets using a single saccharide, reduction in the tongue coating scores was not substantially observed. On the other hand, regarding the tablets of the above-mentioned Example 2, reduction in the tongue coating scores of about 0% was observed every time when 1 tablet was taken. Considering the aforementioned results , it is understood that the tablets of the present invention achieve extremely remarkable effects.

(Test examples: Production of tablet and evaluation of surface roughness by solubility test)

(A. Production)

The tablets of Example 1, Example 9, and Comparative Examples 1 to 4 were used.

Further, in the present test examples, tablets of Examples 11 to 17, and Comparative Example 6 were manufactured under the same procedures and conditions as used in Example 1 except that a total of 100 parts by weight of saccharides with a weight ratio shown in Table 18 below were used with respect to 2 parts by weight of Sugar ester B-370F.

(B. Solution test)

Two tablets of Examples 1 and 9 and Comparative Examples 1 to 4 were respectively put into a beaker containing 100 cc of water. The beakers were shaken using a rotary shaker under a condition of amplitude of 20 mm, 60 rpm. The temperature of the water was about 37°C. Tablets were taken out 3 minutes and 7 minutes after the immersion. Moisture was removed. Thereafter, surface roughness $R_a$ ($\mu$m) of the surface of the tablets was measured using a laser microscope (VK-8500, made by Keyence Corporation) using a surface roughness measurement mode.

(Table 18)

| Tablet | Used Saccharide (Solubility at 37°C (weight %)) | | | Difference of the solubilities between H and L | Weight ratio of H:L:O | Surface roughness $R_a$($\mu$m) | |
|---|---|---|---|---|---|---|---|
| | Highly soluble saccharide (H) | Lowly soluble saccharide (L) | Other saccharide (O) | | | after 3 minutes | after 7 minutes |
| Example 1 | sorbitol (74) | palatinit (37) | maltitol (63) | 37 | 40:32:26 | 37.52 | 78.29 |
| Example 9 | maltitol (63) | palatinit (37) | none (-) | 26 | 37:60:0 | 24.80 | 57.35 |
| Example 11 | fructose (84) | mannitol (23) | none (-) | 61 | 10:90:0 | 54.58 | 105.32 |
| Example 12 | sorbitol (74) | lactose (24) | none (-) | 50 | 25:75:0 | 31.40 | 36.13 |
| Example 13 | xylitol (72) | palatinose (40) | none (-) | 32 | 40:60:0 | 40.57 | 45.94 |
| Example 14 | lactitol (70) | erythritol (44) | none (-) | 26 | 55:45:0 | 33.53 | 58.18 |
| Example 15 | sucrose (68) | maltose (52) | none (-) | 16 | 70:30:0 | 28.32 | 36.33 |
| Example 16 | glucose (67) | trehalose (52) | none (-) | 15 | 85:15:0 | 28.23 | 29.86 |
| Example 17 | fructose (84) | sorbitol (74) | none (-) | 10 | 50:50:0 | 59.00 | 59.26 |
| Comparative Example 1 | glucose (67) | none (-) | none(-) | 0 | 100:0:0 | 12.02 | 13.20 |
| Comparative Example 2 | maltitol (63) | none (-) | none (-) | 0 | 100:0:0 | 12.97 | 14.30 |
| Comparative Example 3 | sorbitol (74) | none (-) | none (-) | 0 | 100:0:0 | 12.41 | 21.27 |

(continued)

| Tablet | Used Saccharide (Solubility at 37°C (weight %)) | | | Difference of the solubilities between H and L | Weight ratio of H:L:O | Surface roughness $R_a(\mu m)$ | |
|---|---|---|---|---|---|---|---|
| | Highly soluble saccharide (H) | Lowly soluble saccharide (L) | Other saccharide (O) | | | after 3 minutes | after 7 minutes |
| Comparative Example 4 | palatinit (37) | none (-) | none(-) | 0 | 100:0:0 | 7.07 | 7.25 |
| Comparative Example 6 | sorbitol (74) | xylitol (72) | none (-) | 2 | 50:50:0 | 13.34 | 19.54 |

As a result, it was understood that sufficient surface roughness is obtained when a difference of solubilities of the saccharides at 37°C is 10 % by weight or more.

For tablets of Examples 1 and 11 to 17, actual measurement of the tongue coating scores was not performed. However, since they have surface roughness similar to that of the tablet of Example 9 which has been demonstrated to have the effect of removing tongue coating, it is understood that these tablets achieve the effect of removing tongue coating.

As mentioned above, the present invention is exemplified as mentioned above using preferable embodiments of the present invention. However, these embodiments should not be construed as limiting the scope of the present invention. It is understood that the scope of the present invention will be interpreted by the appended claims. Those skilled in the art will understand that from detailed preferable embodiments of the present invention, equivalent scope can be carried out based on the description of the present invention and common technical practice. It is understood that contents of patents, patent applications, and literatures cited herein should be incorporated for reference as well as being described in detail herein.

INDUSTRIAL APPLICABILITY

With the use of the tablet for tongue coating removal of the present invention in addition to a physical means such as a tongue brush, an excellent effect of removing tongue coating is obtained. The tablet for removing tongue coating of the present invention has such advantages that the tongue surface is not damaged and nausea is not induced. The tablet for removing tongue coating of the present invention is eatable and is appropriate for use in the oral cavity. Further, the tablet for removing tongue coating of the present invention has further advantages in that it achieves a sufficient effect of removing tongue coating and it can be simply used.

**Claims**

1. A tablet for removing tongue coating, wherein
the tablet contains a highly soluble saccharide and a lowly soluble saccharide,
the highly soluble saccharide and the lowly soluble saccharide are water-soluble,
the highly soluble saccharide is a saccharide having the highest solubility in water at 37°C among the water-soluble saccharides contained in the tablet as the main components,
the lowly soluble saccharide is a saccharide having the lowest solubility in water at 37°C among the water-soluble saccharides contained in the tablet as the main components, and
the difference between the solubility of the highly soluble saccharide in water at 37°C and the solubility of the lowly soluble saccharide in water at 37°C is 10 % by weight or more.

2. A tablet for removing tongue coating, wherein
the tablet contains a highly soluble saccharide and a lowly soluble saccharide,
the highly soluble saccharide and the lowly soluble saccharide are water-soluble,
the highly soluble saccharide is a saccharide having the highest solubility in water at 37°C among the water-soluble saccharides present in the tablet at 10 % by weight or more,
the lowly soluble saccharide is a saccharide having the lowest solubility in water at 37°C among the water-soluble saccharides present in the tablet at 10 % by weight or more, and

the difference between the solubility of the highly soluble saccharide in water at 37°C and the solubility of the lowly soluble saccharide in water at 37°C is 10 % by weight or more.

3. A tablet for removing tongue coating, wherein
the tablet contains a highly soluble saccharide and a lowly soluble saccharide,
the highly soluble saccharide and the lowly soluble saccharide are water-soluble,
the highly soluble saccharide is a saccharide having the highest solubility in water at 37°C among the water-soluble saccharides present in the tablet at 20 % by weight or more,
the lowly soluble saccharide is a saccharide having the lowest solubility in water at 37°C among the water-soluble saccharides present in the tablet at 20 % by weight or more, and
the difference between the solubility of the highly soluble saccharide in water at 37°C and the solubility of the lowly soluble saccharide in water at 37°C is 10 % by weight or more.

4. The tablet according to Claim 3, wherein said difference is 15 % by weight or more.

5. The tablet according to Claim 3, wherein said difference is 20 % by weight or more.

6. The tablet according to Claim 3, wherein said difference is 25 % by weight or more.

7. The tablet according to Claim 3, wherein said difference is 30 % by weight or more.

8. The tablet according to Claim 3, wherein said difference is 35 % by weight or more.

9. The tablet according to Claim 3, wherein the solubility of said highly soluble saccharide in water at 37°C is 50 % by weight or more.

10. The tablet according to Claim 9, wherein said highly soluble saccharide is selected from the group consisting of glucose, maltitol, sorbitol, xylitol, erythritol, maltose, sucrose, fructose, and cellobitol.

11. The tablet according to Claim 3, wherein the solubility of said lowly soluble saccharide in water at 37°C is 5 % by weight or more and 40 % by weight or less.

12. The tablet according to Claim 11, wherein said lowly soluble saccharide is selected from the group consisting of palatinit, mannitol, lactose, and palatinose.

13. The tablet according to Claim 3, further containing another water-soluble saccharide in addition to said highly soluble saccharide and said lowly soluble saccharide.

14. The tablet according to Claim 3, wherein said highly soluble saccharide is sorbitol, said lowly soluble saccharide is palatinit, and said another water-soluble saccharide is maltitol.

15. The tablet according to Claim 3, wherein the tablet has a disintegration time of 5 minutes or more.

16. The tablet according to Claim 3, wherein the tablet has a hardness of 15 kgf or more.

17. The tablet according to Claim 3, further comprising a binder.

18. The tablet according to Claim 3, further comprising an enzyme.

19. The tablet according to Claim 18, wherein said enzyme is a protease.

20. The tablet according to Claim 18, wherein said enzyme is bromelain or papain.

21. A production method of a tablet for removing tongue coating, comprising the steps of:

mixing a highly soluble saccharide and a lowly soluble saccharide to obtain a mixture; and
compressing the mixture to obtain a tablet, wherein
the highly soluble saccharide and the lowly soluble saccharide are water-soluble,

the highly soluble saccharide is a saccharide having the highest solubility in water at 37°C among the water-soluble saccharides contained in the tablet as the main components,
the lowly soluble saccharide is a saccharide having the lowest solubility in water at 37°C among the water-soluble saccharides contained in the tablet as the main components, and
the difference between the solubility of the highly soluble saccharide in water at 37°C and the solubility of the lowly soluble saccharide in water at 37°C is 10% by weight or more.

22. A production method of a tablet for removing tongue coating, comprising the steps of:

mixing a highly soluble saccharide and a lowly soluble saccharide to obtain a mixture; and
compressing the mixture to obtain a tablet, wherein
the highly soluble saccharide and the lowly soluble saccharide are water-soluble,
the highly soluble saccharide is a saccharide having the highest solubility in water at 37°C among the water-soluble saccharides present in the tablet at 10 % by weight or more,
the lowly soluble saccharide is a saccharide having the lowest solubility in water at 37°C among the water-soluble saccharides present in the tablet at 10 % by weight or more, and
the difference between the solubility of the highly soluble saccharide in water at 37°C and the solubility of the lowly soluble saccharide in water at 37°C is 10 % by weight or more.

23. A production method of a tablet for removing tongue coating comprising the steps of:

mixing a highly soluble saccharide and a lowly soluble saccharide to obtain a mixture; and
compressing the mixture to obtain a tablet, wherein
the highly soluble saccharide and the lowly soluble saccharide are water-soluble,
the highly soluble saccharide is a saccharide having the highest solubility in water at 37°C among the water-soluble saccharides present in the tablet at 20 % by weight or more,
the lowly soluble saccharide is a saccharide having the lowest solubility in water at 37°C among the water-soluble saccharides present in the tablet at 20 % by weight or more, and
the difference between the solubility of the highly soluble saccharide in water at 37°C and the solubility of the lowly soluble saccharide in water at 37°C is 10 % by weight or more.

**Fig.1**

**Fig.2**

(A) (B)

*Fig.3*

(A) (B)

*Fig.4*

(A) (B)

Fig.5

(A) (B)

Fig.6

(A) (B)

**Fig.7**

**Fig.8**

**Fig.9**

(A)

Reduction ratio(%)

Example 4 (Bromelain 0.5%)
Example 5 (Papain 0.35%)
Example 6 (Papain 0.25%)
Example 3 (Enzyme-free)

1 tablet    2 tablets    3 tablets

(B)

Reduction ratio(%)

Comparative example 1
Comparative example 2
Comparative example 3
Comparative example 4

1 tablet    2 tablets    3 tablets

Fig.10

*Fig.11*

*Fig.12*

## INTERNATIONAL SEARCH REPORT

| International application No. |
|---|
| PCT/JP2006/317088 |

| A. CLASSIFICATION OF SUBJECT MATTER |
|---|
| *A61K8/60*(2006.01)i, *A61J3/10*(2006.01)i, *A61K9/20*(2006.01)i, *A61K47/26*(2006.01)i, *A61P1/02*(2006.01)i, *A61Q11/00*(2006.01)i |

According to International Patent Classification (IPC) or to both national classification and IPC

| B. FIELDS SEARCHED |
|---|
| Minimum documentation searched (classification system followed by classification symbols) |
| A61K8/00-8/99, A61K9/00-9/72, A61K47/00-47/48, A61Q11/00 |

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| Y | WO 2003/090704 A1 (EZAKI GLICO CO., LTD.), 06 November, 2003 (06.11.03), Example 4, 5; page 38, line 14 to page 40, line 16 & AU 2003231395 A1 & JP 2003-587343 A | 1-23 |
| Y | WO 99/18936 A1 (SS PHARM CO.), 22 April, 1999 (22.04.99), Full text & EP 1022021 A1 & CN 1281356 A & KR 2001024464 A & US 6455053 B1 & TW 527195 A | 1-23 |

| ☒ Further documents are listed in the continuation of Box C. | ☐ See patent family annex. |
|---|---|

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 November, 2006 (28.11.06) | 12 December, 2006 (12.12.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
|---|
| PCT/JP2006/317088 |

C (Continuation).  DOCUMENTS CONSIDERED TO BE RELEVANT

| Category* | Citation of document, with indication, where appropriate, of the relevant passages | Relevant to claim No. |
|---|---|---|
| A | WO 95/20380 A1  (YAMANOUCHI PHARM CO., LTD.),<br>03 August, 1995 (03.08.95),<br>Full text<br>& AU 9514671 A          & ZA 9500702 A<br>& FI 9603022 A          & JP 07-519980 A<br>& NO 9603180 A          & US 5576014 A<br>& EP 745382 A1          & KR 97700488 A<br>& NZ 278678 A           & MX 9603035 A1<br>& CN 1139878 A          & TW 391880 A<br>& PH 31467 A | 1-23 |
| A | WO 2002/92057 A1  (ASTELLAS PHARMA INC.),<br>21 November, 2002 (21.11.02),<br>Full text<br>& US 2003/099701 A1     & EP 1323417 A1<br>& NO 200300947 A        & BR 200205709 A<br>& EP 1374858 A2         & CN 1473036 A<br>& KR 2004002839 A       & AU 2002255321 A1<br>& JP 2002-588975 A      & AU 2002255321 A2<br>& MX 2003001838 A1      & US 2005/100599 A1 | 1-23 |
| A | WO 2003/009831 A1  (YAMANOUCHI PHARM CO.,<br>LTD.),<br>06 February, 2003 (06.02.03),<br>Full text<br>& BR 200205509 A        & US 2003/147948 A1<br>& KR 2003036656 A       & HU 200302351 A2<br>& CN 1473035 A          & EP 1413294 A1<br>& AU 2002355222 A1      & ZA 200300647 A<br>& JP 2003-515224 A      & MX 2003000985 A1<br>& TW 200412245 A | 1-23 |
| A | WO 2002/32403 A1  (DAIICHI PHARM CO., LTD.),<br>25 April, 2002 (25.04.02),<br>Full text<br>& AU 200194250 A        & NO 200301622 A<br>& EP 1334716 A1         & KR 2003042006 A<br>& US 2004/014680 A1     & JP 2002-535641 A<br>& CN 1469737 A | 1-23 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03090704 A

**Non-patent literature cited in the description**

- **TONZETICH J.** Direct gas chromatographic analysis of sulphur compounds in mouth air in man. *Archs. Oral Biol.,* 1971, vol. 16, 587-597
- **TONZETICH J. ; COIL J.M. ; NG W.** Gas chromatographic method for trapping and detection of volatile organic compounds from human mouth air. *J. Clin. Dent.,* 1991, vol. 11, 79-82
- **TONZETICH J. ; EIGAN E. ; KING W.J. et al.** Volatility as a factor in the inability of certain amines and indole to increase the odour of saliva. *Archs. Oral Biol.,* 1967, vol. 12, 1167-1175
- **TONZETICH J. ; MCBRIDE B.C.** Characterization of volatile sulfur production by pathogenic and non-pathogenic strains of oral Bacteroides. *Archs. Oral Biol.,* 1981, vol. 26, 963-969
- **YAEGAKI KEN ; MIYAZAKI HIDEO ; KAWAGUCHI YOKO.** Rinsho-Ka No Tame No Koshu Chiryo Guideline. Quintessence Publishing, 2000
- **YAEGAKI K. ; SANAGA K.** Biochemical and clinical factors influencing oral malodor in periodontal patients. *J. Periodontol.,* 1992, vol. 63, 783-789
- **MORIYA TOSHIKI ; KISHI MITSUO ; AIZAWA FUMIE et al.** Zettai Score Ni Yoru Koshu Screening No Yukosei Ni Kansuru Kenkyu. *Journal of Dental Health,* 2002, vol. 52, 12-21